(19) **Europäisches Patentamt / European Patent Office / Office européen des brevets**

(11) **EP 3 139 339 A1**

(12) **EUROPEAN PATENT APPLICATION**
published in accordance with Art. 153(4) EPC

(43) Date of publication:
08.03.2017 Bulletin 2017/10

(21) Application number: 15785990.1

(22) Date of filing: 20.02.2015

(51) Int Cl.:
*G06Q 50/22* (2012.01)    *A61B 5/00* (2006.01)
*G06Q 50/24* (2012.01)    *G06T 1/00* (2006.01)

(86) International application number:
PCT/JP2015/054703

(87) International publication number:
WO 2015/166692 (05.11.2015 Gazette 2015/44)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 30.04.2014 JP 2014093475

(71) Applicant: Konica Minolta, Inc.
Tokyo 100-7015 (JP)

(72) Inventor: MATSUDA Shinya
Tokyo 100-7015 (JP)

(74) Representative: Alton, Andrew
Urquhart-Dykes & Lord LLP
Arena Point
Merrion Way
Leeds LS2 8PA (GB)

(54) **HEALTH DEGREE ASSESSING DEVICE AND HEALTH DEGREE ASSESSING SYSTEM**

(57)    A health degree assessing device (terminal device (51)) which assesses a degree of health of a subject is provided with: a feature quantity input unit (communication unit (54)) which accepts as inputs feature quantities which are extracted from a captured image of an organ of the subject and are used to assess the degree of health of the subject; and an assessing unit (53) which compares the feature quantities of the subject, input by way of the feature quantity input unit, with the distribution of the feature quantities of a population, to assess deviations in the physical constitution of the subject corresponding to individual differences with other individuals, and changes in the physical condition of the subject. When assessing deviations in the physical constitution of the subject, the assessing unit (53) uses, as the distribution of the feature quantities of the population, the distribution of the feature quantities of the other individuals, prepared using the other individuals as the target, and when assessing changes in the physical condition of the subject, the assessing unit (53) uses, as the distribution of the feature quantities of the population, the distribution of the feature quantities of the subject himself or herself, collected during an evaluation period in which the physical condition of the subject is being evaluated.

FIG.16

## Description

### Technical Field

The present invention relates to a level-of-health determining device and a level-of-health determining system for determining an examinee's level of health.

### Background Art

Conventionally, there have been known, as methods for measuring the health condition of a living body (e.g., a human), biochemical examination in medical checkups, statement of subjective complaints on medical questionnaires, physical fitness measurement, etc. However, medical checkups take cost and time, and are unsuitable for the monitoring of day-to-day change of physical condition. Moreover, while medical checkups allow checks for the presence of particular diseases, due to segmentalized diagnostic items, it is difficult to describe an overall health condition.

Medical questionnaires are filled in with subjective complaints, and thus involve large individual differences; this makes a comparison with past histories or with other people difficult. Like medical checkups, physical fitness measurement takes cost and time, and is unsuitable for the monitoring of day-to-day change of physical condition. Moreover, although physical fitness measurement allows the measurement of athletic ability, no definite correlation is known between physical strength and health condition. Thus, it is doubtful whether physical fitness measurement helps grasp health condition.

There has thus recently been proposed a system in which an image of the tongue is taken with a digital camera, and based on characteristic quantities from the image, level of health is diagnosed. For example, according to Patent Document 1, a single kind of output information (specifically, a Mahalanobis distance) is calculated from a number of characteristic quantities obtained from a tongue image, and thereby an overall determination of level of health of the person the image of whose tongue bas been taken can be made easily and objectively. A system that makes an overall determination of level of health by use of a Mahalanobis distance is also proposed, for example, in Patent Document 2.

### List of Citations

### Patent Literature

Patent Document 1: Japanese Patent No. 4487535, Publication (see claim 1, paragraphs 0006, 0018, 0096; etc.)
Patent Document 2: Japanese Patent No. 4649965, Publication (see claim 1, paragraph 0008; etc.)

### Summary of the Invention

### Technical Problem

Due to hereditary factors and long-term lifestyles, the condition of the tongue is subject to individual differences (including age differences) even in good health. That is, even in good health, some people naturally have a bluish tongue color, some other people a reddish tongue color, and so forth. Accordingly, simply by determining level of health by extracting the color of the tongue as a characteristic quantity from taken images of the tongue, it is not possible to distinguish whether a bluish tongue color is due to a disparity of physical constitution (individual difference) or a change of physical condition (level of health). As a result, a determination of level of health can be made only as including a disparity of physical constitution; that is, it is not possible to determine level of health accurately. Also, because of lack of information on a disparity of physical constitution, it is difficult to confirm the effect of long-term improvement plans such as alimentary therapies and improvements in lifestyle. Such and similar problems arise even with the techniques disclosed in Patent Documents 1 and 2, where multivariate analysis is performed using a Mahalanobis distance.

Devised to solve the problems discussed above, the present invention aims to provide a level-of-health determining device and a level-of-health determining system which permit accurate determination of level of health (health condition) while reducing the effect of a disparity of physical constitution and which permits easy confirmation of the effect of an effort to control a disparity of physical constitution.

### Means for Solving the Problem

According to one aspect of the present invention, a level-of-health determining device includes: a characteristic quantity acceptor to which a characteristic quantity extracted from a taken image of an organ of an examinee to be used for level-of-health determination is input; and a determiner which compares the examinee's characteristic quantity input via the characteristic quantity acceptor with a distribution of a characteristic quantity of a population and which thereby determines a disparity of the examinee's physical constitution, which corresponds to an individual difference from other people, and a change of the examinee's physical condition. Here, when determining the disparity of the examinee's physical constitution, the determiner uses, as the distribution of the population's characteristic quantity, a distribution of a characteristic quantity of the other people that is created with the other people taken as a target. On the other hand, when determining the change of the examinee's physical condition, the determiner uses, as the distribution of the population's characteristic quantity, a distribution of the examinee's own characteristic quantity collected during an evaluation period for physical condition evaluation.

## Advantageous Effects of the Invention

With the configuration described above, it is possible to accurately determine level of health (health condition) while reducing the effect of a disparity of physical constitution, and to easily confirm the effect of an effort to control a disparity of physical constitution.

## Brief Description of Drawings

Fig. 1 is an explanatory diagram showing an outline configuration of a level-of-health determining system according to one embodiment of the present invention;
Fig. 2 is a perspective view showing an exterior appearance of an organ imaging device provided in the above level-of-health determining system;
Fig. 3 is a block diagram showing an outline configuration of the above organ imaging device;
Fig. 4 is an explanatory diagram showing a positional relationship between an illuminator and an imager with respect to an imaging target;
Fig. 5 is an explanatory diagram showing an example of a taken image of a tongue, an example of an edge extraction filter, and a contour line of the tongue extracted by the filter;
Fig. 6 is an explanatory diagram showing a region where a characteristic quantity is extracted from a taken image of a tongue;
Fig. 7 is a graph showing a contour line of a tongue and an approximate curve of the contour line;
Fig. 8 is a graph plotting differences in y-coordinate between the above contour line and the above approximate curve;
Fig. 9 is an explanatory diagram showing a taken image of a tongue and sectional shapes of the tongue;
Fig. 10 is an explanatory diagram showing regions for detection of degree of gloss in the above taken image;
Fig. 11 is a graph showing a spectrum distribution on a tongue;
Fig. 12 is a graph schematically showing a frequency distribution of B image data extracted from the above detection regions;
Fig. 13 is an explanatory diagram showing a taken image of a tongue with fissures on the surface;
Fig. 14 comprises graphs showing frequency distributions, one for a tongue with no fissures on the surface and the other with fissures on the surface;
Fig. 15 comprises graphs showing distributions of image data in the horizontal direction, one for a thin tongue and the other for a thick tongue;
Fig. 16 is a block diagram showing an outline configuration of a terminal device provided in the above level-of-health determining system;
Fig. 17 is an explanatory diagram schematically showing a distribution of other people's characteristic quantities created by a determiner in the terminal device;
Fig. 18 is a graph showing an image of long-term change of physical condition with a patient, a healthy subject, and an examinee;
Fig. 19 is a graph showing, on an enlarged scale, change of physical condition during a given evaluation period with the examinee in Fig. 18;
Fig. 20 is a flow chart showing a flow of operation in the above level-of-health determining system;
Fig. 21 is a graph showing change of an evaluation value (first evaluation value) of an examinee's physical condition;
Fig. 22 is a graph showing, on an enlarged scale, change of the evaluation value of the examinee's physical condition during the evaluation period in Fig. 21;
Fig. 23 is a graph showing change of an evaluation value (second evaluation value) of an examinee's physical condition;

Fig. 24 is a graph showing medium-term change of an evaluation value (first evaluation value) of an examinee's physical condition; and

Fig. 25 is a graph showing a frequency distribution with a characteristic quantity composed of a single variable.

**Description of Embodiments**

An embodiment of the present invention will be described below with reference to the accompanying drawings. In the present description, any numerical range from the value A to the value B is assumed to include the lower limit A and the upper limit B.

[Overall Configuration of a Level-of-Health Determining System]

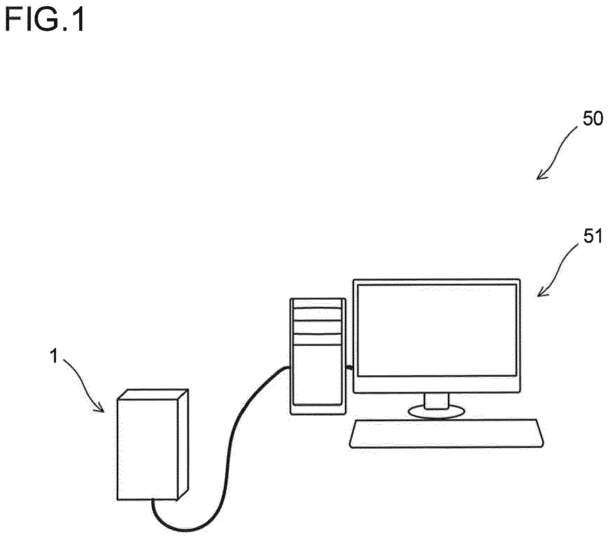

Fig. 1 is an explanatory diagram showing an outline configuration of a level-of-health determining system 50 according to one embodiment of the present invention. The level-of-health determining system 50 includes an organ imaging device 1 and a terminal device 51. The organ imaging device 1 takes an image of an organ of a living body to extract (detect) information (characteristic values, items to be diagnosed, etc.) needed to diagnose the level of health, and comprises, for example, a multi-function portable information terminal. The terminal device 51 determines the level of health of the living body based on the information acquired by the organ imaging device 1, and comprises a personal computer or the like.

The organ imaging device 1 and the terminal device 51 are connected together across a communication network so as to be capable of communicating with each other. The communication network may be wired or wireless. At least part of the functions of the terminal device 51 may be implemented in the organ imaging device 1; reversely, at least part of the functions of the organ imaging device 1 may be implemented in the terminal device 51. The organ imaging device 1 and the terminal device 51 will now be described in detail. The following description assumes a human as a living body and the tongue as an organ.

[Configuration of the Organ Imaging Device]

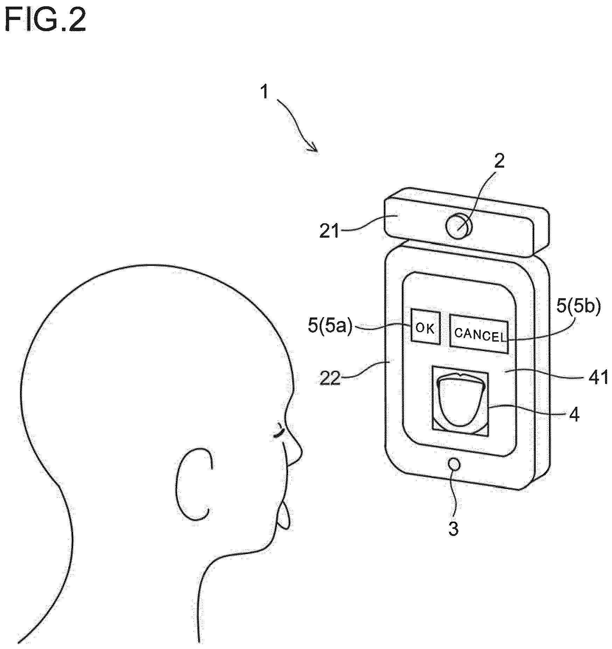

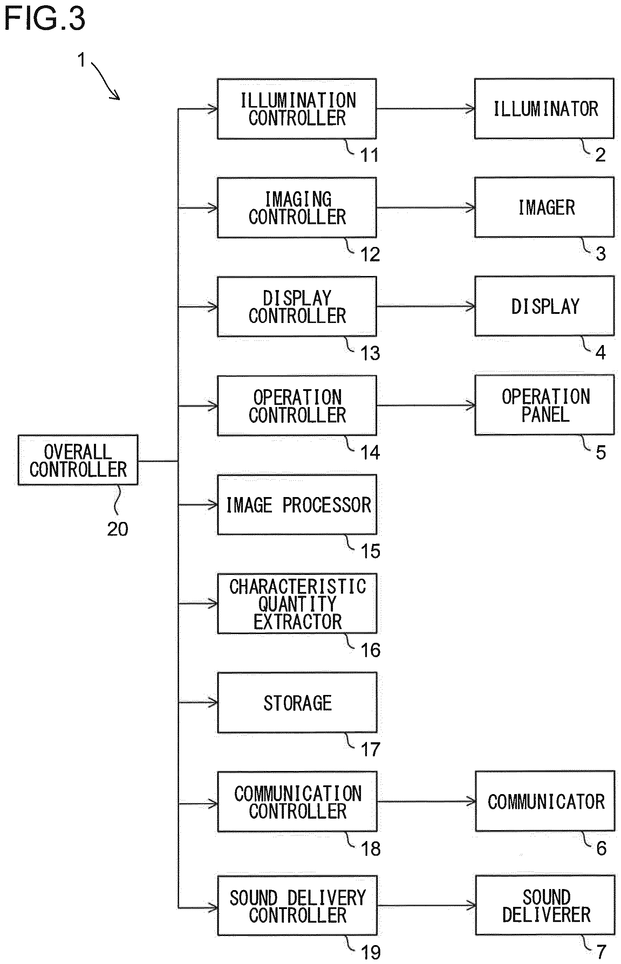

Fig. 2 is a perspective view showing an exterior appearance of the organ imaging device 1 according to the embodiment, and Fig. 3 is a block diagram showing an outline configuration of the organ imaging device 1. The organ imaging device 1 includes an illuminator 2, an imager 3, a display 4, an operation panel 5, a communicator 6, and a sound deliverer 7. The illuminator 2 is provided in a housing 21, and the blocks other than the illuminator 2 (e.g., the imager 3, the display 4, the operation panel 5, the communicator 6, and the sound deliverer 7) are provided in a housing 22. The housings 21 and 22 are coupled together so as to be rotatable relative to each other, but do not necessarily have to be so: one of them may be completely secured to the other. The illuminator 2 and the other blocks mentioned above may be provided in a single housing.

The illuminator 2 serves to illuminate an organ (here, the tongue) of a living body as an imaging target, and comprises a lamp that illuminates the imaging target from above. The illuminator 2 includes a light source that emits light of a daylight color, such as a xenon lamp, for improved color rendering. The brightness of the light source varies depending on the sensitivity of the imager 3 and the distance to the imaging target; the brightness can be, for example, such as to provide an illumination of 1000 to 10000 1x at the imaging target. The illuminator 2 includes, in addition to the light source, a lighting circuit and a dimming circuit, and is controlled according to instructions from an illumination controller 11 so as to be lit, extinguished, and dimmed.

The imager 3 serves to image an organ (here, the tongue) of a living body to acquire an image of it under the illumination of the illuminator 2, and includes an imaging lens and an area sensor (image sensor). The aperture of the imaging lens (the fastness of the lens), the shutter speed, and the focal length are so set that the imaging target is in focus over its entire area. For example, the f-number can be set at 16, the shutter speed can be set at 1/120 seconds, and the focal length can be set at 20 mm.

The area sensor comprises, for example, an image sensor such as a CCD (charge-coupled device) image sensor or a CMOS (complementary metal oxide semiconductor) image sensor, and its sensitivity, resolution, etc. are so set that the color and shape of the imaging target can be detected satisfactorily. For example, the sensitivity can be set at 60 dB, and the resolution can be set at 10 megapixels.

The imaging by the imager 3 is controlled by an imaging controller 12. The imager 3 further includes, in addition to the imaging lens and the area sensor, a focusing mechanism, an aperture mechanism, a drive circuit, an A/D conversion circuit, etc., of which none is illustrated, and is controlled according to instructions from the imaging controller 12 in terms of focus, aperture, A/D conversion, etc. The imager 3 acquires, as the data of a taken image, data comprising, for example, eight bits, representing a value from 0 to 255, for each of red (R), green (G), and blue (B) per pixel.

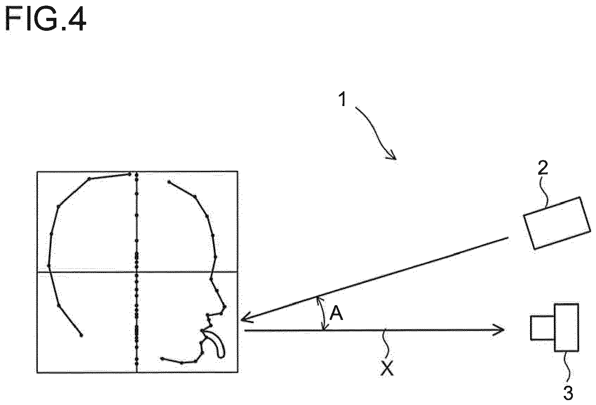

Fig. 4 is an explanatory diagram showing a positional relationship of the illuminator 2 and the imager 3 with respect to an imaging target (the tongue or the face). As shown there, the imager 3 is arranged right in front of the imaging target. The illuminator 2 is so arranged as to illuminate the imaging target, for example, at an angle A of 0° to 45° relative to the imaging optical axis X of the imager 3, which passes through the imaging target. The imaging optical axis X denotes the optical axis of the imaging lens provided in the imager 3.

Here, when illumination is applied at a large angle A, the shadow of the upper lip reduces the area over which the tongue can be imaged. Reversely, when illumination is applied at a small angle A, specular reflection causes severe color clipping. Out of these considerations, a preferred range of the angle A for illumination is from 15° to 30°.

The display 4 includes a liquid crystal panel, a backlight, a lighting circuit, and a control circuit, of which none is illustrated, and displays the image acquired by the imaging by the imager 3 and information calculated and output by a characteristic quantity extractor 16, which will be described later. The display 4 can also display items needed for a diagnostic interview, which will be described later, and information (e.g., the result of diagnosis performed at an external medical facility based on information transmitted to it) acquired from outside via the communicator 6. The displaying of various kinds of information on the display 4 is controlled by a display controller 13.

The operation panel 5 comprises an input device from which to instruct the imager 3 to perform imaging, and includes an OK button (TAKE IMAGE button) 5a and a CANCEL button 5b. In this embodiment, the display 4 and the operation panel 5 are constituted by a single touch panel display device 41, and separate display regions are provided on the touch panel display device 41, one for the display 4 and the other for the operation panel 5. The displaying of the operation panel 5 on the touch panel display device 41 is controlled by an operation controller 14. The operation panel 5 may be configured as any other input device than the touch panel display device 41 (the operation panel 5 may be provided anywhere else than inside the display region of the touch panel display device 41).

The operation panel 5 also functions as an information input device for answering items needed for a diagnostic interview that are displayed on the display 4. In a case where, as described above, the display 4 and the operation panel 5 are constituted by a touch panel display device 41, the display 4 displays such items along with keys (e.g., a numerical keypad, a shift key, an OK button, etc.) to be operated by an operator to answer them, so that the operator can enter necessary information via the displayed keys. Thus, in this case, the display 4 doubles as the above-mentioned information input device in the operation panel 5.

The communicator 6 comprises an interface for transmitting the data of the image acquired by the imager 3 and the information calculated and output by the characteristic quantity extractor 16, which will be described later, to the outside via a communication network, and for receiving information from the outside. In particular, the communicator 6 constitutes a transmitter that transmits the information on characteristic quantities of an examinee as extracted by the characteristic quantity extractor 16 to a terminal device 51. The transmission and reception of information by the communicator 6 is controlled by a communication controller 18.

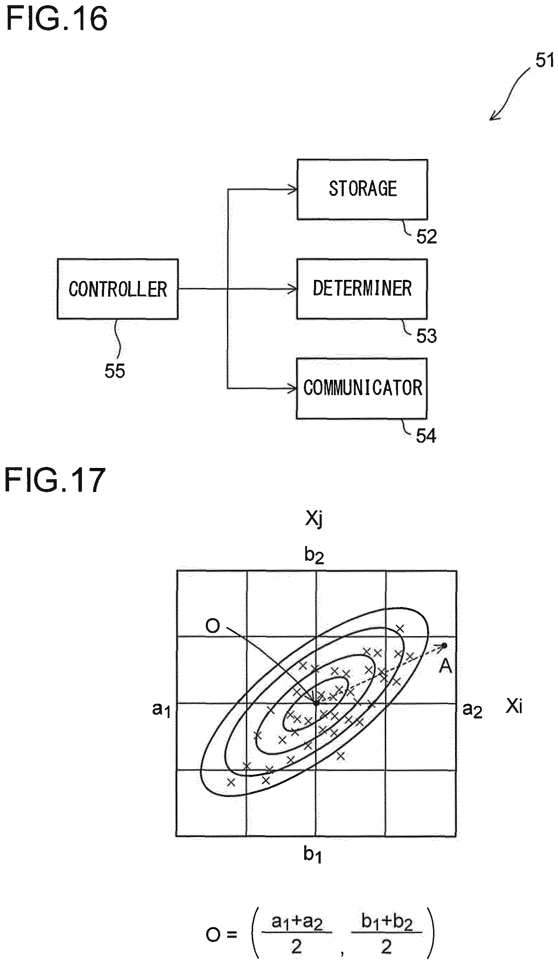

The sound deliverer 7 outputs various kinds of information in the form of sound, and comprises, for example, a speaker. Information that is output in the form of sound includes a result of evaluation by a determiner 53 (see Fig. 16), which will be described later, provided in the terminal device 51. The sound delivery from the sound deliverer 7 is controlled by a sound delivery controller 19.

The organ imaging device 1 further includes an illumination controller 11, an imaging controller 12, a display controller 13, an operation controller 14, an image processor 15, a characteristic quantity extractor 16, a storage 17, a communication controller 18, a sound delivery controller 19, and an overall controller 20 which controls those blocks. As mentioned above, the illumination controller 11, the imaging controller 12, the display controller 13, the operation controller 14, the communication controller 18, and the sound delivery controller 19 control the illuminator 2, the imager 3, the display 4, the operation panel 5, the communicator 6, and the sound deliverer 7 respectively. The overall controller 20 comprises, for example, a CPU (central processing unit). The illumination controller 11, the imaging controller 12, the display controller 13, the operation controller 14, the communication controller 18, and the sound delivery controller 19 may be configured integrally with the overall controller 20 (e.g., as a single CPU).

The image processor 15 extracts a contour line of an organ from the image acquired by the imager 3. In this embodiment, the image processor 15 extracts the contour line of the tongue as an organ based on a luminance edge in the taken image (a part of the image where luminance changes sharply).

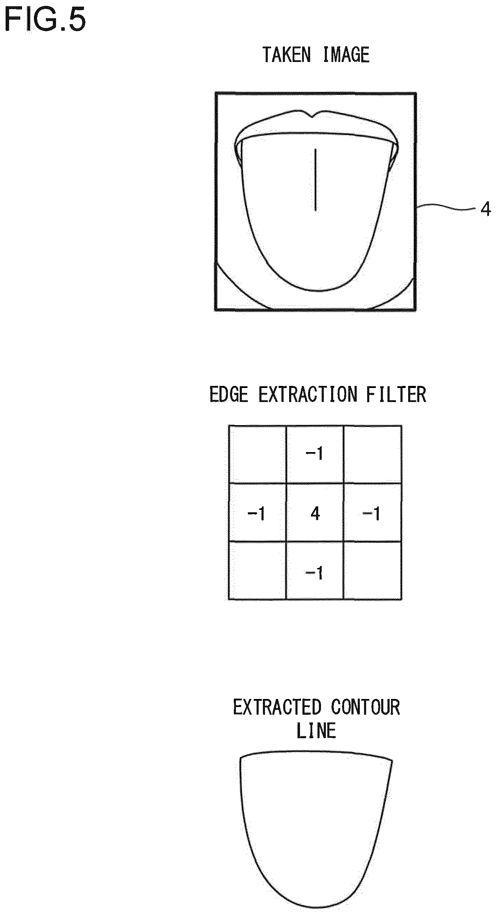

A luminance edge can be extracted, for example, by use of an edge extraction filter as shown in Fig. 5. An edge extraction filter is a filter that gives weights to pixels near a pixel of interest when performing first-order differentiation (when calculating differences in image data between neighboring pixels). By use of such an edge extraction filter, for example, with respect to the green image data of each pixel in the taken image, differences in image data are calculated between the pixel of interest and neighboring pixels, and those pixels which yield differences exceeding a predetermined threshold value are extracted; in this way, pixels that constitute a luminance edge can be extracted. Around the tongue, its shadow produces brightness differences; thus, by extracting pixels that constitute a luminance edge in the manner described above, it is possible to extract the contour line of the tongue. Here, green image data is used in the calculation because it has the greatest influence on luminance; red or blue image data may be used instead.

The storage 17 comprises a memory that stores the data of the image acquired by the imager 3, the data acquired by the image processor 15, the data calculated by the characteristic quantity extractor 16, the data received from the outside, etc., and also stores programs for operating the various controllers mentioned above.

The characteristic quantity extractor 16 is a processor or circuit that extracts, from the image data acquired by the imager 3, characteristic quantities that will be used for evaluation of the level of health. The characteristic quantities include the color of the tongue, the color of the tongue coating (called "tongue moss" in some schools of oriental medicine), the shape (thickness) of the tongue coating, tooth marks, moistness (the degree of gloss), a fissure pattern (cracks), and the shape (thickness) of the tongue. These characteristic quantities can be extracted (detected), for example, in the following manners.

(Extracting Tongue Color, Tongue coating Color, and Tongue Coating Thickness)

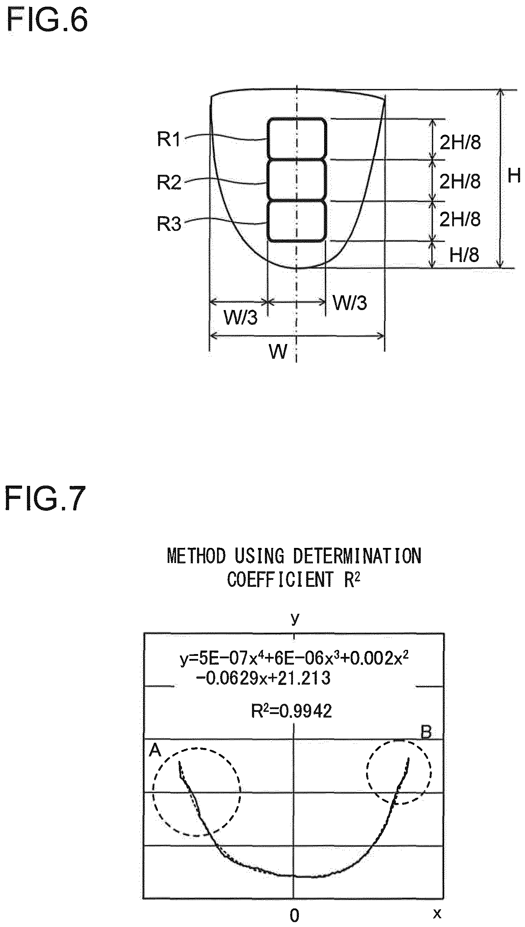

Fig. 6 shows a taken image of a tongue. The following description deals with a case where, in the taken image of the tongue, a strip-form region that extends in the up-down direction in a middle part of the tongue in the left/right direction is divided into three regions that are referred to as an upper region R1, a middle region R2, and a lower region R3 respectively. These regions are defined by the dimensions shown in Fig. 6 based on the width W in the left/right direction and the length H in the up-down direction of the region surrounded by the contour line of the tongue detected by the image processor 15. The illustrated dimensions, however, are merely examples, and are not meant as any limitation.

The color of the tongue reflects the color of blood; thus, in RGB image data, chiefly an R component or a B component exhibits an increase or a decrease. Accordingly, by finding the proportion of R, i.e., $(R / (R + G + B))$, or the proportion of B, i.e., $(B / (R + G + B))$, in the lower region R3 of the tongue, it is possible to quantify and extract the color of the tongue. As the RGB data mentioned above, it is possible to use the average value of R image data, the average value of G image data, and the average value of B image data among the plurality of pixels that compose the lower region R3.

The reason that, for extraction of the tongue color, the image data in the lower region R3 is used is as follows: in tongue diagnosis practiced in *Kampo* (the Japanese adaptation of traditional Chinese medicine), the color of the tongue is inspected in left and right end parts of the tongue that have no tongue coating or in a lower central part of the tongue; this is because left and right end parts of the tongue tend to be illuminated differently due to surface irregularities and thus with varying brightness, causing deviations of image data from those values that show the natural color of the tongue.

On the other hand, the tongue coating exhibits a white to brown color depending on the amount of cornfield tissues; thus, in RGB image data, chiefly a G component or a (G + R) component exhibits an increase or a decrease. Accordingly, by finding the proportion of G, i.e., $(G / (R + G + B))$, or the proportion of (G + R), i.e., $((G + R) / (R + G + B))$, in the upper region R1 of the tongue, it is possible to quantify and extract the color of the tongue coating. As the RGB data mentioned above, it is possible to use the average value of R image data, the average value of G image data, and the average value of B image data among the plurality of pixels that compose the upper region R1.

The reason that, for extraction of the tongue coating color, the image data in the upper region R1 is used is that the tongue coating forms through cornification of papilla tissues in the mucous membrane on the tongue and it forms over an upper to a middle part of the tongue, in particular in an upper part.

As a tongue coating becomes thicker, the color changes from red, the inherent color of the tongue, to white, the color of the tongue coating; thus, in RGB image data, chiefly an R or G component exhibits an increase or a decrease. Accordingly, by finding the proportion of R, i.e., $(R / (R + G + B))$, or the proportion of G, i.e., $(G / (R + G + B))$, in the middle region R2 of the tongue, it is possible to quantify and extract the thickness of the tongue coating. As the RGB data mentioned above, it is possible to use the average value of R image data, the average value of G image data, and the average value of B image data among the plurality of pixels that compose the middle region R2.

The reason that, for extraction of the tongue thickness, the image data in the middle region R2 is used is that, as mentioned above, a tongue coating forms in an upper part of the tongue and thus, based on whether or not the color in the middle region R2 is close to that in the upper region R1, the thickness of the tongue coating can be determined. The tongue coating thickness may also be quantified by finding the ratio of the difference in color (e.g., the difference in chromaticity) between the upper and middle regions R1 and R2 to the difference in color (e.g., the difference in chromaticity) between the middle and lower regions R2 and R3.

(Extracting Tooth Marks)

The characteristic quantity extractor 16 approximates the tongue's contour line acquired by the image processor 15 to an approximate curve, detects irregularities (smoothness) in the contour line based on the degree of correlation between the contour line and the approximate curve, and thereby detects tooth marks. The approximate curve is a smooth curve with no irregularities, and thus it can be said that, the closer the contour line is to the approximate curve, the smoother the contour line is, and the fewer tooth marks it has.

Accordingly, based on the degree of correlation, it is possible to detect the condition of tooth marks. For example, a high degree of correlation can be evaluated as indicating a mild case of tooth marks (level 1), a low degree of correlation can be evaluated as indicating a severe case of tooth marks (level 3), and a medium degree of correlation somewhere between can be evaluated as indicating a medium case of tooth marks between mild and severe (level 2),

Here, as an index that indicates the degree of correlation between the two lines, one of the following three indices, referred to as a first to a third index respectively, can be used.

A first index that indicates the degree of correlation is a determination coefficient $R^2$ given by the formula below.

$$R^2 = 1 - \{(\Sigma(yi - fi)^2) / (\Sigma(yi - Y)^2)\}$$

where

i represents a value between j and k, with j and k representing the x coordinates of one and the other ends, respectively, of the contour line or the approximate curve on the xy plane;
yi represents the y coordinate of the point on the contour line at the x coordinate i on the xy plane;
fi represents the y coordinate of the point on the approximate curve at the x coordinate i on the xy plane; and
Y represents the average value of yi for all points on the contour line.

Here, i, j, and k are all integers, fulfilling $j < k$ and $j \le i \le k$. $\Sigma(yi - fi)^2$ represents the sum of $(yi - fi)^2$ as i is varied from j to k, and $\Sigma(yi - Y)^2$ represents the sum of $(yi - Y)^2$ as i is varied from j to k.

Fig. 7 shows s tongue contour line (indicated by a solid line), an approximate curve (indicated by a broken line) that approximates it, the polynomial equation that expresses the approximate curve, and the determination coefficient $R^2$. The approximate curve is found by the least-square method, and is expressed by the polynomial equation below. Here, the determination coefficient $R^2$ equals 0.9942. For any coefficient in the approximate curve shown in Fig. 7, "E-n" stands for "$\times 10^{-n}$".

$$y = 5 \times 10^{-7} \cdot x^4 + 6 \times 10^{-6} \cdot x^3 + 2 \times 10^{-3} \cdot x^2 + 6.29 \times 10^{-2} \cdot x + 21.213$$

Both in part A, with a severe tooth mark (large irregularities in the contour line), and in part B, with a mild tooth mark (small irregularities in the contour line), the differences between the contour line and the approximate curve are reflected in the determination coefficient $R^2$. Accordingly, by use of a method that detects a tooth mark based on the determination coefficient $R^2$, even with only a mild tooth mark, it is possible to detect it based on the determination coefficient $R^2$, leading to improved tooth-mark detection accuracy.

A second index that indicates the degree of correlation is a value obtained based on differences in coordinates (y coordinates) between the contour line and the approximate curve, and is, specifically, the maximum value of $|yi - fi|$ (hereinafter also referred to as the "maximum value of coordinate differences").

Here,

i represents a value between j and k, with j and k representing the x coordinates of one and the other ends, respectively, of the contour line or the approximate curve on the xy plane;
yi represents the y coordinate of the point on the contour line at the x coordinate i on the xy plane; and
fi represents the y coordinate of the point on the approximate curve at the x coordinate i on the xy plane.

Here, i, j, and k are all integers, fulfilling $j < k$ and $j \le i \le k$.

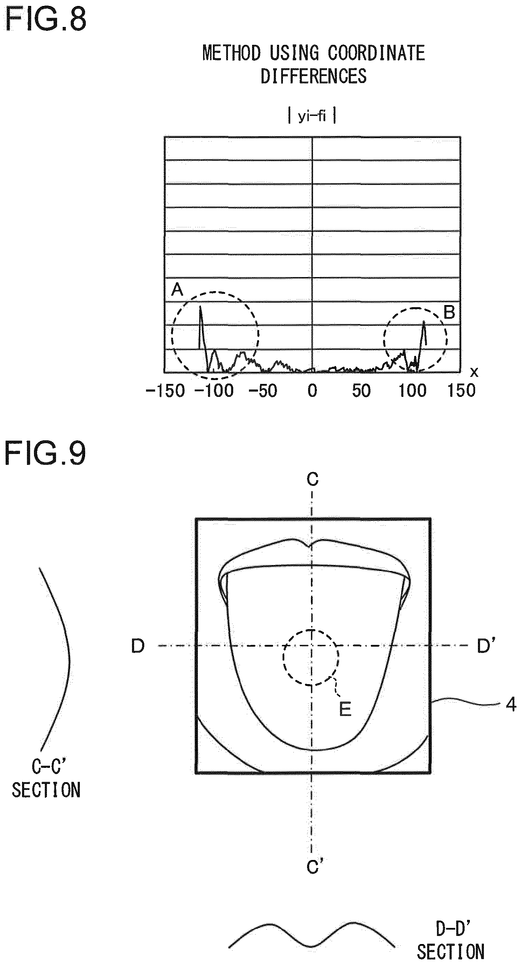

Fig. 8 is a graph that plots y-coordinate differences ($|yi - fi|$) between the tongue contour line and its approximate curve (an xy polynomial equation) shown in Fig. 7. Both in part A, with a severe tooth mark, and in part B, with a mild tooth mark, the value of $|yi - fi|$ is greater than elsewhere, with no tooth mark. Thus, even with only a mild tooth mark, it is possible to detect it, by detecting the maximum value of $|yi - fi|$, based on this value, leading to improved tooth-mark detection accuracy.

A third index that indicates the degree of correlation is a value obtained based on differences in coordinates (y coordinates) between the contour line and the approximate curve, and is a coefficient A given by the formula below.

$$A = \Sigma|yi - fi|$$

where

i represents a value between j and k, with j and k representing the x coordinates of one and the other ends, respectively, of the contour line or the approximate curve on the xy plane;

yi represents the y coordinate of the point on the contour line at the x coordinate i on the xy plane; and

fi represents the y coordinate of the point on the approximate curve at the x coordinate i on the xy plane.

Here, i, j, and k are all integers, fulfilling $j < k$ and $j \leq i \leq k$. $\sum|yi - fi|$ represents the sum of $|yi - fi|$ as i is varied from j to k (hereinafter also referred to as the "sum of coordinate differences").

Using the sum of coordinate differences instead of the maximum value of coordinate differences allows tooth-mark detection that accurately reflects the influence of a fine tooth mark, leading to further improved tooth-mark detection accuracy.

As the degree of correlation, it is also possible to use the reciprocal of the sum of the coordinate differences, namely $1 / (\sum|yi - fi|)$.

(Extracting Moistness (the Degree of Gloss))

Fig. 9 shows a taken image of a tongue along with sectional shapes of the tongue. When the tongue is imaged, it is protracted out of the oral cavity. To permit the imager 3 to image the upper lip-side surface of the protracted tongue, this surface is curved so as to be convex toward the imager 3 (see the C-C' section). As necessary, how to protract the tongue can be explained in a specification or instruction manual so that the examinee can hold the tongue in a proper imaging position.

When the tongue is imaged with the illuminator 2 and the imager 3 arranged as shown in Fig. 4, a specular reflection region appears in the upper half of the tongue (because the illuminator 2 is located above the imaging optical axis X). On the other hand, in the left/right direction with respect to the tongue, a middle part of the tongue and the left and right ends of the tongue sag to describe an M-shape (see the D-D' section). The tongue has a largely similar sectional shape from an upper to a lower part of it. Moreover, in a central part E of the tongue, a pattern of cracks may be observed. Accordingly, in the embodiment, illumination is applied at an angle of 15 degrees, and a region on the upper half of the tongue excluding a middle part and opposite end parts of it in the left/right direction is set as a region suitable for the detection of the degree of gloss.

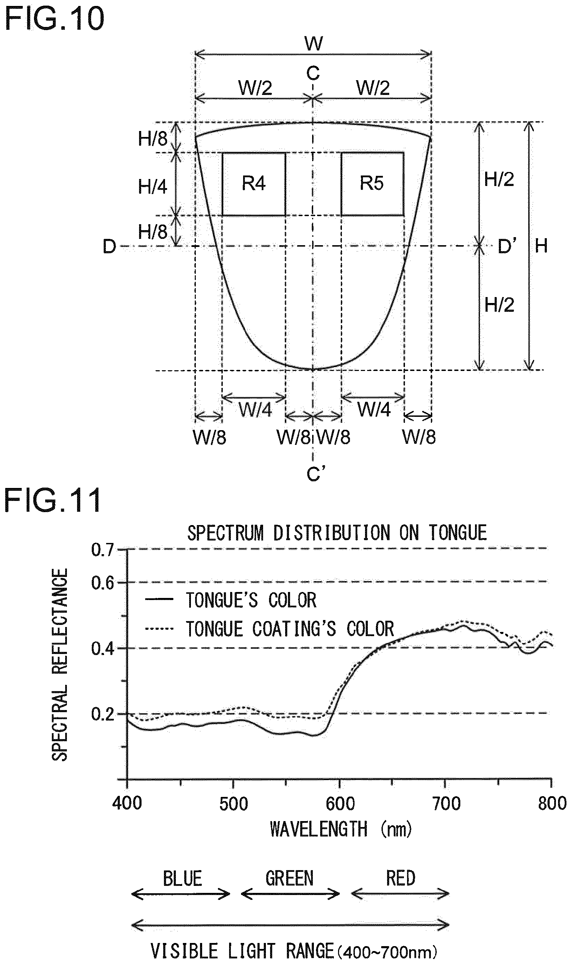

More specifically, from the contour line of the tongue detected by the image processor 15, the characteristic quantity extractor 16 detects the top, bottom, left, and right ends of the tongue to detect the top-to-bottom length H and the left-to-right width W of the tongue. Then, with reference to the contour line of the tongue, the characteristic quantity extractor 16 sets degree-of-gloss detection regions R4 and R5 such that they have a positional relationship as shown in Fig. 10.

Fig. 11 is a graph showing a spectrum distribution on the tongue. The tongue has a mucous membrane structure without epidermis, and its color reveals the color of blood. In the color of blood, the proportion of an R component (with wavelengths from 600 nm to 700 nm) is high, and the proportion of a B component (with wavelengths of 500 nm or less) is low. A lighter tongue color yields a lower proportion of the R component, and a darker tongue color yields a higher proportion of the R component.

On the other hand, the tongue coating is formed of cornfield cells of papillae, and takes on a white to yellow color. With a thin tongue coating, the color of the tongue beneath prevails, and thus the proportion of the R component is high, as shown in Fig. 11. With a white, thick tongue coating, the proportion of the G component (with wavelengths from 500 nm to 600 nm) is high.

While the colors of the tongue and the tongue coating vary as described above with the health condition of the living body and differences among individuals, the proportion of the B component varies little. Accordingly, in the embodiment, the degree of gloss on the surface of the tongue is detected based on the B image data obtained from a taken image of the tongue through a procedure as described below.

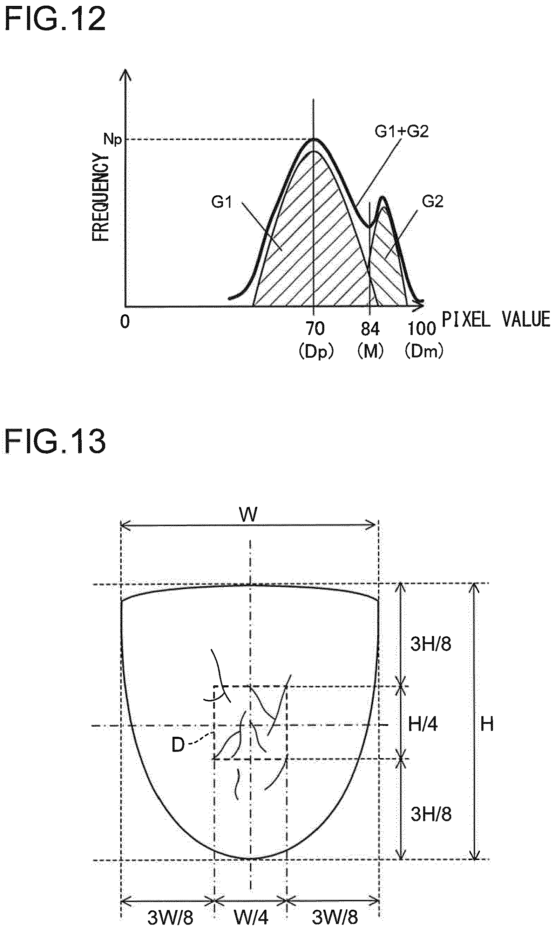

First, the characteristic quantity extractor 16 extracts B image data from pixels in the degree-of-gloss detection regions R4 and R5 of the taken image, and creates a frequency distribution of the data. Fig. 12 schematically shows a frequency distribution of the extracted B image data. In Fig. 12, the horizontal axis represents the B pixel value (image data), and the vertical axis represents the frequency (the number of pixels). Here, however, for simplicity's sake, it is assumed that a pixel value takes a value from 1 to 100, a greater pixel value indicating higher luminance.

Next, from the above-mentioned frequency distribution, the characteristic quantity extractor 16 finds the pixel value Dp that corresponds to the highest frequency Np (in the example in Fig. 12, Dp = 70). The characteristic quantity extractor 16 then multiplies that pixel value Dp by 1.2 to calculate the threshold value M (in the example in Fig. 12, M = 84). Then, over the range from the threshold value M to the maximum value of the image data (the maximum pixel value Dm = 100), the characteristic quantity extractor 16 cumulates the frequencies to calculate the number of high-value pixels. The pixel value Dp may instead be calculated by first finding a function that continuously represents how the frequency varies, then smoothing and removing noise from the function, and then calculating the pixel value Dp corresponding to the highest frequency Np. The number of high-value pixels may be found by integrating the smoothed function over a predetermined range.

Here, if no specular reflection occurs on the tongue surface during imaging, the frequency distribution of the B image data is composed of a single distribution close to a normal distribution (a first group G1). By contrast, if specular reflection occurs, the first group G1 is accompanied by another distribution (a second group G2) with high frequencies at higher pixel values. In addition, since the B image data varies little with the health condition of the living body and differences among individuals as mentioned above, the width of the first group G1 (the width from the minimum to the maximum pixel value in the first group G1) is smaller than the frequency distribution (normal distribution) of other image data, such as of R or G. As a result, a clear border between the first and the second groups G1 and G2 (a point at which the frequency has a minimum, turning from decrease to increase) appears between the value of image data at which the frequency is highest (i.e., the pixel value Dp) and the maximum value of image data (i.e., the maximum pixel value Dm). Thus, the first and the second groups G1 and G2 can be distinguished from each other easily. In detecting the degree of gloss, it is preferable to do so not based on the first group G1, which includes no gloss component (specular reflection component), but based on the second group G2, which represents the gloss component.

Accordingly, the characteristic quantity extractor 16 sets the threshold value M greater than the pixel value Dp, and finds the sum of frequencies between the threshold value M and the maximum pixel value Dm as the number of high-value pixels, so as to obtain a value close to the sum of frequencies in the second group G2.

In particular, it has been experimentally found out that, in a frequency distribution of B image data, the boundary between the first and the second groups G1 and G2 appears in the range from 1.1 to 1.3 times the pixel value Dp. Accordingly, in the embodiment, the characteristic quantity extractor 16 sets the threshold value M within the range from 1.1 to 1.3 times the pixel value Dp (in the example in Fig. 12, 1.2Dp = 84), and finds the sum of frequencies between the threshold value M and the maximum pixel value Dm as the number of high-value pixels.

It has been found that, in a case where the tongue is dry (with a low degree of gloss), the number of high-value pixels is low and, in a case where the tongue is moist (with a high degree of gloss), the number of high-value pixels is high. Accordingly, based on the number of high-value pixels, the characteristic quantity extractor 16 can detect the degree of gloss in a digitized fashion, for example on a scale from level 1 (a high degree of gloss) to level 3 (a low degree of gloss).

(Extracting Fissures (Cracks))

Fig. 13 shows a taken image of a tongue with fissures on the surface. When the tongue is imaged, it is protracted out of the oral cavity. The upper lip-side surface of the protracted tongue is imaged by the imager 3. Generally, fissures on the tongue surface develop predominantly at and around the center of the tongue. Accordingly, in the embodiment, a central part of the tongue in the up-down and left-right directions (a region including its center in the up-down and left-right directions) is set as a region suitable for the detection of fissures.

More specifically, from the contour line of the tongue detected by the image processor 15, the characteristic quantity extractor 16 detects the top, bottom, left, and right ends of the tongue to detect the top-to-bottom length (longitudinal dimension) and the left-to-right width (lateral dimension) W of the tongue. The characteristic quantity extractor 16 then sets, as a fissure detection region D, a central region of the tongue with a longitudinal dimension of H/4 and a lateral dimension of W/4 determined based on the dimensional relationship shown in Fig. 13.

With fissures on the tongue surface, more of the tongue's underlayer is exposed than with no fissures on the tongue surface; this widens the ranges of values that the image data of pixels corresponding to the underlayer can take, for all of R, G, and B. Accordingly, when a frequency distribution of the image data of the taken image is created, it has a larger frequency distribution width. In particular, the underlayer strongly reflects the color of blood; thus, the R and B components, of which high proportions are included in the color of blood, have notably wider frequency distribution widths than with no fissures. It has been found that this tendency is observed irrespective of the thickness of the tongue coating or the length of cracks on the tongue surface.

Accordingly, in the embodiment, the characteristic quantity extractor 16 creates a frequency distribution of, for example, B image data from the taken image of the tongue (in particular, the image of the detection region D), and in addition calculates and acquires, as an index that indicates the spread of the frequency distribution, a standard deviation $\sigma$ which indicates the dispersion of the image data. In a case where image data has N values consisting of $x_1$, $x_2$, ... and $x_N$, the standard deviation $\sigma$ is the positive square root of the dispersion $\sigma^2$ given by the following formula.

[Formula 1]

$$\sigma^2 = \frac{1}{N} \sum_{i=1}^{N} (x_i - m)^2$$

where

$$m = \frac{1}{N} \sum_{i=1}^{N} x_i$$

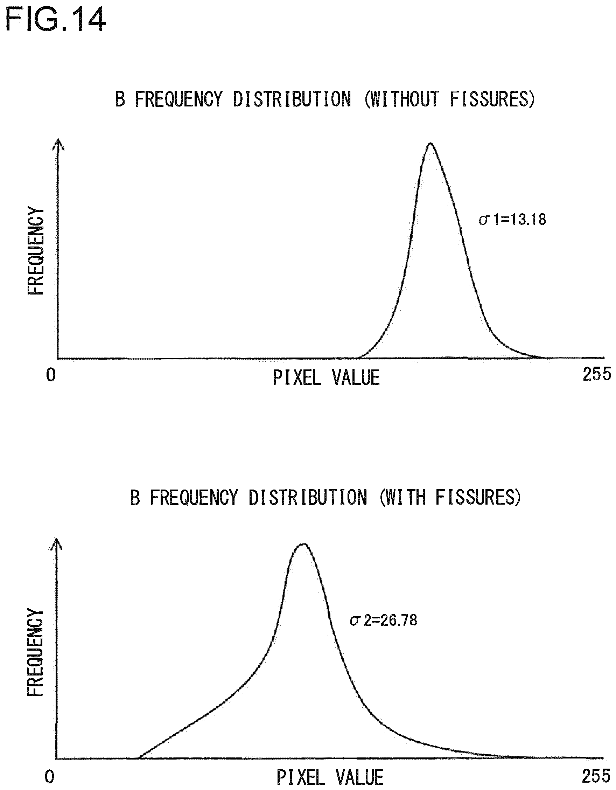

Fig. 14 schematically shows frequency distributions of B image data created by the characteristic quantity extractor 16, the frequency distribution at top depicting a case where no cracks are observed on the tongue surface, the frequency distribution at bottom depicting a case where cracks are observed on the tongue surface. In these frequency distributions, the horizontal axis represents B pixel value (image data) and the vertical axis represents frequency (number of pixels). The pixel value takes a value from 0 to 255, a larger value representing higher luminance.

Finding the standard deviation $\sigma 1$ in the frequency distribution at top gives $\sigma 1$ = 13.18. On the other hand, finding the standard deviation $\sigma 2$ in the frequency distribution at bottom gives $\sigma 2$ = 26.78. These results reveal that, with cracks on the tongue surface, the standard deviation $\sigma$ is higher than with no cracks, and the frequency distribution has a larger width. Incidentally, finding the average value m1 of pixel values in the frequency distribution at top gives m1 = 177.71, and finding the average value m2 of pixel values in the frequency distribution at bottom gives m2 = 112.75.

It has been found that a case involving many cracks yields a high standard deviation in the above frequency distribution and a case involving few cracks yields a low standard deviation in the above frequency distribution. Accordingly, based on the standard deviation of the above frequency distribution, the characteristic quantity extractor 16 can detect cracks in a digitized fashion, for example on a scale from level 1 (many cracks) to level 3 (few cracks). Although the example above deals with a case where cracks are detected based on the frequency distribution of B image data, it is also possible to detect cracks based on the frequency distribution of other, i.e., R or G, image data.

(Extracting Tongue Thickness)

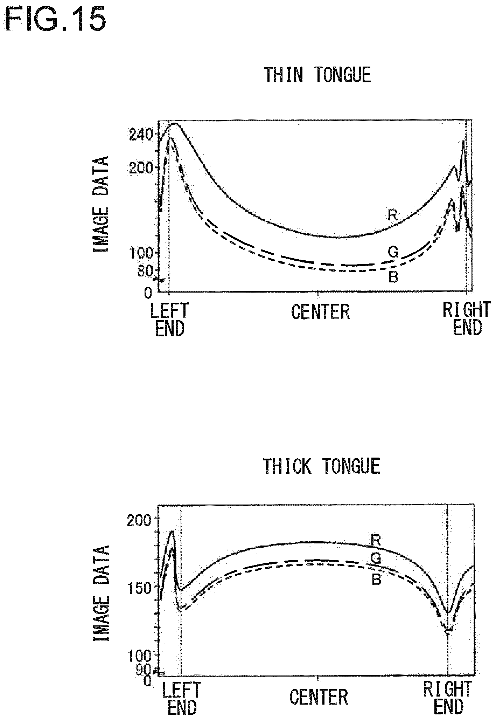

Fig. 15 shows distributions of image data obtained when the tongue surface is imaged by the imager 4, showing distributions of RGB image data of taken images in the horizontal direction across a line passing approximately the center of the tongue surface in the up-down direction. The distribution at top depicts a case where the tongue is thin, and the distribution at bottom depicts a case where the tongue is thick. A solid line represents the distribution of R image data, a dash-and-dot line represents the distribution of G image data, and a broken line represents the distribution of B image data.

A thick tongue includes, from an end part to a middle part thereof, a part that is convex upward. The convex part on the tongue surface is closer to the illuminator 2, and is illuminated brighter. Thus, in a part of a taken image of the tongue corresponding to the convex part, image data has increased values. On the other hand, a thin tongue includes, from an end part to a middle part thereof, a part that is largely flat or concave downward. The flat or concave part on the tongue surface is farther away from the illuminator 2 than the convex part mentioned above, and appears dimmer than the convex part even when illuminated. Thus, in a part of a taken image of the tongue corresponding to the flat or concave part on the surface, image data has decreased values. This tendency is observed similarly for all of R, G, and B image data.

Accordingly, the characteristic quantity extractor 16 can detect whether the tongue is thick or thin based on irregularities in the distribution of image data in the horizontal direction for one of R, G, and B image data (the distribution of one color) in a taken image of the tongue obtained under the illumination of the illuminator 2. Specifically, by using the distribution of image data in the horizontal direction for one of R, G, and B included in a taken image of the tongue as a data distribution that indicates irregularities on the tongue surface, and quantifying the degree of irregularities, for example, on a scale from level 1 (thick tongue) to level 5 (thin tongue), it is possible to detect the tongue thickness accurately (irrespective of the exterior shape of the tongue). The degree of irregularities in a data distribution can be discriminated by calculating an approximate curve (e.g., a quadratic equation) that approximates a central part of the data distribution by the least-square method and inspecting the quadratic coefficient of the approximate curve and its absolute value. Incidentally, a positive quadratic coefficient, the approximate curve has a concave shape; with a negative quadratic coefficient, it has a convex shape.

As the above-mentioned distribution of image data, a distribution of data representing the R component proportion (R / (R + G + B)), the G component proportion (G / (R + G + B)), or the B component proportion (B / (R + G + B) may instead be used, even in which case the tongue thickness can be detected accurately.

(Other)

Characteristic quantities for the tongue shape, tooth marks, a fissure pattern, etc. can be extracted by the method disclosed in Patent Document 1. As disclosed in Patent Document 2, a taken image of the tongue may be divided into a plurality of regions so that RGB data is read out on a region-by-region basis to extract characteristic quantities.

[Terminal Device]

Next, the terminal device 51 will be described. Fig. 16 is a block diagram showing an outline configuration of the terminal device 51. The terminal device 51 is a level-of-health determining device that evaluates the level of health of an examinee, and includes a storage 52, a determiner 53, a communicator 54, and a controller 55.

The storage 52 is a memory that stores the data of taken images of an organ (here, the tongue) of a plurality of other people and the data transmitted from external devices including the organ imaging device 1. The communicator 54 is an interface or terminal that conducts communication with an external device. In particular, in this embodiment, the communicator 54 constitutes a characteristic quantity acceptor that is fed with characteristic quantities extracted from a taken image of an organ of an examinee and used for evaluation of the level of health, and constitutes a receiver that receives information on characteristic quantities of the examinee transmitted from the outside (the organ imaging device 1). The terminal device 51 may further include a reader that reads characteristic quantities from a recording medium on which characteristic quantities extracted from a taken image of an organ of the examinee are recorded. In this case, the terminal device 51 can acquire characteristic quantities by reading information in the reader. Accordingly, in this case, the reader can be operated to function as the characteristic quantity acceptor mentioned above. The controller 55 comprises, for example, a CPU, and controls different parts of the terminal device 51.

The determiner 53 is a processor or circuit that compares a characteristic quantity of an examinee transmitted from the organ imaging device 1 and received via the communicator 54 serving as a characteristic quantity acceptor with the distribution of the characteristic quantity in a population, and thereby determines a disparity of the examinee's physical constitution, which corresponds to an individual difference from others, and a change of the examinee's physical condition. Here, as a distribution of a characteristic quantity in a population, it is possible to use the distributions of the characteristic quantity of other people or the distribution of the characteristic quantity of the examinee himself. These distributions are created by the determiner 53. Specifically, the distribution of a characteristic quantity of other people is created, by an extraction method similar to that in the characteristic quantity extractor 16, by extracting the characteristic quantity from the data of taken images of other people stored in the storage 52. The distribution of a characteristic quantity of an examinee is created by use of the characteristic quantity extracted by the characteristic quantity extractor 16 during an evaluation period for evaluation of physical condition.

Fig. 17 schematically shows a distribution, created by the determiner 53, of characteristic quantities of other people. In the diagram, each x-mark indicates the plot position of a characteristic quantity of one other individual. Here, as an example, a distribution of two-dimensional characteristic quantities (Xi, Xj) is shown. Xi can be, for example, the color of the tongue, and Xj can be, for example, the color of the tongue coating. The symbols $a_1$ and $a_2$ along the horizontal axis represent values corresponding to different colors, and likewise the symbols $b_1$ and $b_2$ along the vertical axis represent values corresponding to different colors. The origin O of the distribution of these characteristic quantities is the point that indicates the averages of Xi and Xj.

In the illustrated example, it is seen that the distributions of Xi and Xj are biased and have a correlation between them. Specifically, for Xi values that are located on the $a_1$ side of the origin O, more Xj values are found on the $b_1$ side than on the $b_2$ side, and for Xi values that are located on the $a_2$ side of the origin O, more Xj values are found on the $b_2$ side than on the $b_1$ side. It is considered that, also for three- or more dimensional characteristic quantities, they exhibit a certain correlation with respect to one another.

Here, in Fig. 17, the distance from the origin O of the distribution of the characteristic quantities of other people to the examinee's entered characteristic quantities (e.g., point A) is taken as a physical condition evaluation value (first evaluation value). This evaluation value is not an Euclidean distance but a Mahalanobis distance. A Mahalanobis distance is a measure of distance that is calculated with consideration given to the dispersion of each of many measurement items (characteristic quantities) and the correlation among them. Let the number of members of a population be n, the average of data of each measurement item be m, the standard deviation be $\sigma$, the member of the inverse matrix with respect to the correlation matrix representing the correlation among measurement items be aij, and the number of measurement items be k, then the average value vector of m1, m2, ... mk is the origin of the distribution (space) formed by the population. Here, let the vectors of the standard deviations be $\sigma1$, $\sigma2$, ... $\sigma$k and the measured values (characteristic quantities) of the examinee whose level of health is to be determined be x1, x2, ... xk, then the Mahalanobis distance is given by Formula 2 below.

[Formula 2]

$$D^2 = \frac{1}{k} \sum_{ij} a_{ij} \left( \frac{x_i - m_i}{\sigma i} \right) \left( \frac{x_j - m_j}{\sigma j} \right)$$

In Formula 2 above, the average of the n Mahalanobis distances that are all the targets in the population space equals "1". This determines the origin and the unit of distance for the measure that indicates the evaluation value of physical condition. The smaller the Mahalanobis distance, the closer the examinee is to the population; the larger the Mahalanobis distance, the farther away the examinee is from the population.

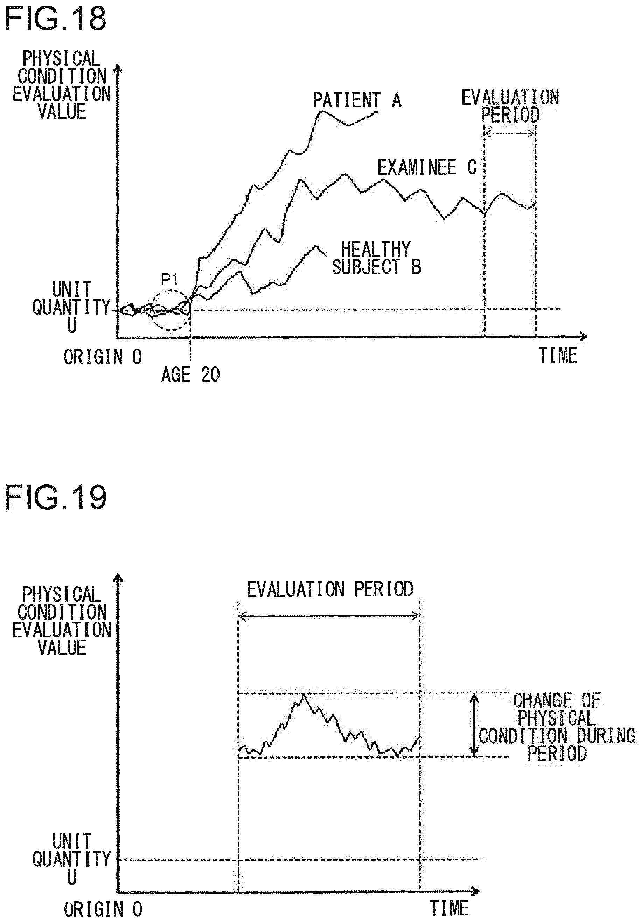

Fig. 18 shows an image of long-term change of physical condition for Patient A as an ill subject, Healthy Subject B, and Examinee C as a target of level-of-health determination. In the diagram, the origin O corresponds to the origin O of the distribution of the characteristic quantities of other people shown in Fig. 17, the horizontal axis representing the time from birth, the vertical axis representing the above-mentioned evaluation value of physical condition. The unit quantity U on the vertical axis indicates the unit of distance from the origin O.

Without a notable disease, little difference in physical condition is observed from birth through the ages of teens for all of Patient A, Healthy Subject B, and Examinee C (with short distances to the origin O) (in the present description, "teens" are assumed to include ages 10 through 19). Over age 20, due to alcohol intake, smoking, irregular lifestyle, etc., individual differences in physical condition start to show, so that even Healthy Subject B may exhibit individual differences (an increased distance to the origin O) such as a weak stomach, a high blood pressure, a disposition to catch cold, etc. Here, such individual differences are defined as "disparities of physical constitution".

On the other hand, Fig. 19 shows, for Examinee C in Fig. 18, change of physical condition during a given evaluation period on an enlarged scale. The same individual experiences day-to-day change of physical condition, such as gastrointestinal discomfort, palpitation, shortness of breath, fever from cold, etc. When any of such conditions deteriorates beyond a limit, it may manifest as a disease that requires treatment. The change of the evaluation value of Examinee C during a given evaluation period is defined as a "change of physical condition". Now, a description will be given of a basic flow of operation including a method of evaluation by the determiner 53.

(Flow of Operation)

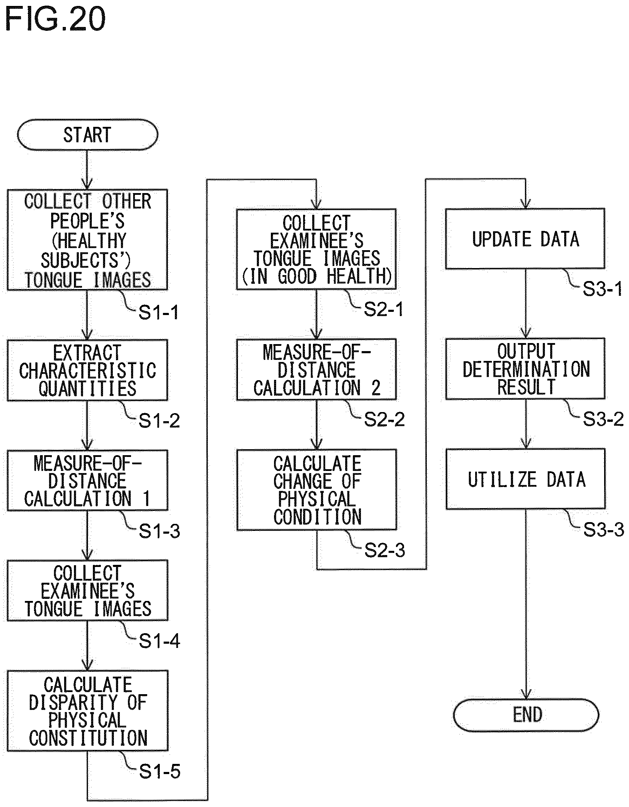

Fig. 20 is a flow chart showing a flow of operation in the level-of-health determining system 50 according to the embodiment. In the level-of-health determining system 50, a first to a third step as described below are performed sequentially.

<1st Step>

(1-1. Collecting Tongue Images of Healthy Subjects)

First, by use of an imaging device (e.g., a digital camera) furnished with a flash, tongue images of young healthy subjects (other people) with no disparity of physical constitution are collected. The healthy subjects belong to the population P1 in Fig. 18. It is believed that the tongue comes to have largely the same appearance as in adults through the ages of teens. Here, images are collected, as samples, from healthy subjects in the first half of their teens with no disparity of physical constitution due to lifestyle, such as adult-onset diseases. Preferably, about 10 to 100 samples are collected. To calculate the Mahalanobis distances mentioned above, more samples need to be collected than there are characteristic quantities (to calculate the inverse matrix). The data of the collected images is transmitted to the terminal device 51 and is stored in the storage 52.

(1-2. Extracting Characteristic Quantities)

The determiner 53 in the terminal device 51 extracts, by an extraction method similar to that in the characteristic quantity extractor 16, characteristic quantities of other people to be used for level-of-health determination from the data of taken images of other people stored in the storage 52. In this way, characteristic quantities for tongue color, tongue coating color, tongue coating shape (thickness), tooth marks, moistness (degree of gloss), fissure pattern (cracks), tongue shape (thickness), etc. are extracted as characteristic quantities of other people. Any other characteristic quantity used for diagnosis in oriental medicine may be extracted.

(1-3. Measure-of-Distance Calculation 1)

Next, the determiner 53 sets the origin $O_1$ and the unit quantity $U_1$ of a distribution that has the extracted characteristic quantities of other people as a population (e.g., with two-dimensional characteristic quantities, a distribution like the one shown in Fig. 17). The origin $O_1$ is the point that indicates the averages of the characteristic quantities of other people, and the unit quantity $U_1$ represents the unit of the distance from the origin $O_1$. These can be set through a multivariate analysis using the above-mentioned Mahalanobis distances. The multivariate analysis may instead use a linear function or a regression analysis, and may be performed by any method.

(1-4. Day-to-Day Image Taking)

By the imager 3 in the organ imaging device 1, day-to-day tongue images of the examinee are collected in a manner similar to that described above. Preferably, twice as many (about 20 to 200) samples are collected. The characteristic quantity extractor 16 extracts characteristic quantities for tongue color, etc. from the data of taken images of the examinee by the method described above. The images taken by the imager 3 and information on the characteristic quantities extracted by the characteristic quantity extractor 16 are transmitted to the terminal device 51 and is stored in the storage 52.

(1-5. Calculating a Disparity of Physical Constitution)

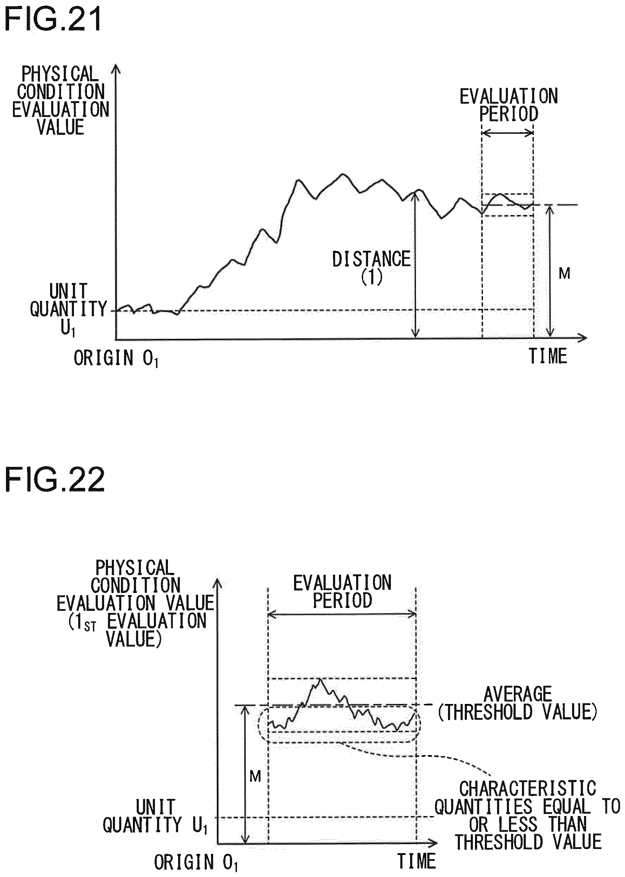

The determiner 53 in the terminal device 51 compares the characteristic quantities of the examinee with the distribution of the population (the distribution of other people's characteristic quantities) to find the difference (Mahalanobis distance) from the population, and finds the change of the Mahalanobis distance with the lapse of time as the change of the evaluation value (first evaluation value) of the examinee's physical condition. Fig. 21 shows the change, thus found, of the evaluation value of the examinee's physical condition. The difference of the examinee's characteristic quantities from the population corresponds to distance (1) in Fig. 21. The determiner 53 finds the average of distance (1) during the evaluation period (as distance M), and determines this distance M as the disparity of the examinee's physical constitution.

<2nd Step>

(2-1. Collecting Examinee's Tongue Images in Good Health)

Fig. 22 shows the change of the evaluation value of the examinee's physical condition during the evaluation period on an enlarged scale. Using the above-mentioned distance M as a threshold value, the determiner 53 extracts images with dates and times (shooting dates) within the evaluation period at which the evaluation value is equal to or less than the threshold value. Here, instead of distance M, a value such as (Average Value - Standard Deviation) may be used as a threshold value so that images with evaluation values equal to or less than the threshold value are extracted (images taken at dates at which the level of health was higher). In that case, preferably, an accordingly increased number of samples are taken.

(2-2. Measure-of-Distance Calculation 2)

Next, the determiner 53 extracts the examinee's characteristic quantities from the examinee's own taken images at all dates and times at which the characteristic quantities were equal to or less than the threshold value, and sets the origin $O_2$ and the unit quantity $U_2$ of a distribution that has the examinee's extracted characteristic quantities as the population. The origin $O_2$ is a point that indicates the averages of the examinee's characteristic quantities, and the unit quantity $U_2$ represents the unit of the distance from the origin $O_2$. These can be set through a multivariate analysis using the above-mentioned Mahalanobis distances. The multivariate analysis may instead be performed by use of a linear function or a regression analysis.

(2-3. Calculating Change of Physical Condition)

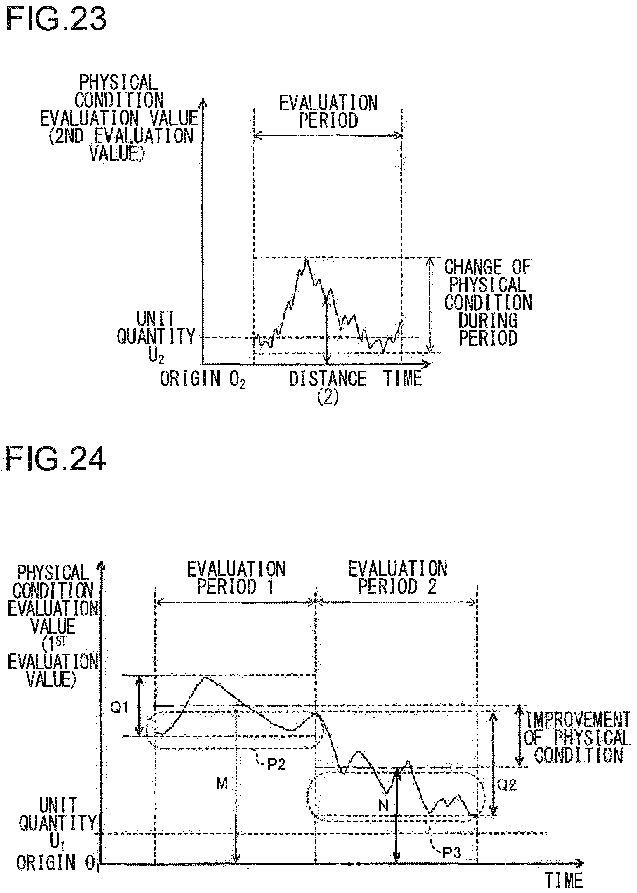

The determiner 53 compares the examinee's characteristic quantities at the time of evaluation with the distribution of the population (the distribution of the examinee's characteristic quantities) to find the difference (Mahalanobis distance) from the population, and determines the change of the Mahalanobis distance with the lapse of time as the change of the evaluation value (a second evaluation value) of the examinee's physical condition. Fig. 23 shows the change of the evaluation value of the examinee's physical condition at that time. Incidentally, the difference between the examinee's characteristic quantities and the population corresponds to distance (2) in Fig. 23. The determiner 53 determines the change of distance (2) during the evaluation period as the change of the examinee's physical condition. Since the distribution of the examinee's own characteristic quantities is taken as the population, no general rise due to a disparity of physical constitution is involved, and thus day-to-day change of physical condition can be measured with satisfactory sensitivity.

<3rd Step>

(3-1. Updating Data)

Fig. 24 shows medium-term change of the evaluation value (first evaluation value) of the examinee's physical condition. When one evaluation period (e.g., evaluation period 1) expires, a transition into the next evaluation period (e.g., evaluation period 2) occurs. On this occasion, the population used for evaluation of the change of physical condition, that is, the distribution of the examinee's characteristic quantities of which the first evaluation value is equal to or less than the threshold value, is updated every predetermined period. In the example shown in Fig. 24, during evaluation period 1, for evaluation of the change of physical condition, population P2 (the distribution of the examinee's characteristic quantities of which the first evaluation value is equal to or less than distance M) is used; on the other hand, during evaluation period 2, population P3 (the distribution of the examinee's characteristic quantities of which the first evaluation value is equal to or less than distance N) is used. Here, distance N is the average of the distance (Mahalanobis distance) of the first evaluation value from origin $O_1$ during evaluation period 2. The population may be updated by replacing all the data at once, or by adding new data and deleting old data on a day-to-day basis. Fig. 24 shows an example of the former method.

During evaluation period 2, using the above-mentioned distance N as a threshold value, the determiner 53 extracts images with shooting dates within evaluation period 2 at which the first evaluation value is equal to or less than the threshold value, and sets the origin $O_2$ and the unit quantity $U_2$ of the distribution of the examinee's characteristic quantities (the distribution of population P3). The determiner 53 then takes the distance between the examinee's characteristic quantities at the time of evaluation during evaluation period 2 with population P3 as the change of the examinee's physical condition during evaluation period 2. In Fig. 24, Q1 indicates the width of the change of physical condition during evaluation period 1, and Q2 indicates the width of the change of physical condition during evaluation period 2. The difference between distances M and N corresponds to an improvement in physical condition from evaluation period 1 to evaluation period 2. The difference from population 2 during the previous evaluation period 1 may be used as the distance from the population in evaluation period 2. Population P1 mentioned previously does not need to be changed.

As population P2 is updated with population P3, distance M is updated with distance N. Likewise, in each subsequent evaluation period, distance N is updated with another. Distance (2) shown in Fig. 23 is updated through day-to-day image taking.

(3-2. Outputting Results)

Information on a change of physical condition and a disparity of physical constitution evaluated during an evaluation period (the value of distance M in Fig. 22 or distance N in Fig. 24, the value of distance (2) in Fig. 23) is transmitted from the terminal device 51 to the organ imaging device 1, and is displayed on the display 4.

(3-3. Utilizing Data)

The terminal device 51 can make a graph of day-to-day variation of the distance and present it to the examinee (organ imaging device 1). If distance (2), which indicates change of physical condition, is increasing and thus the examinee's level of health is deteriorating, or if distance M, which indicates disparity of physical constitution, is large, the terminal device 51 can present an improvement plan as well. The improvement plan so presented may be based on oriental medicine or occidental medicine.

It is also possible to present a specific improvement plan based on the degree of contribution of characteristic quantities. For example, in a case where the tongue color has a large contribution in the degraded level of health, a plan for improving the blood stream can be presented and, in a case where the tongue coating thickness has a large contribution, a plan for improving immunity can be presented.

The determined change of physical condition (distance (2)) may further be compared with another norm to determine whether the physical condition is good or bad. In that case, the examinee's own history may be used. For example, if a tongue image taken when the examinee was ill in the past is available, it can be transmitted to the terminal device 51 so that, in the terminal device 51, characteristic quantities are extracted from the tongue image, the distance from the origin of the population (the distribution of characteristic quantities of other people) at that time is found (in a case where a plurality of tongue images taken when the examinee was ill are available, the average of such distances), and the result can be used as the above-mentioned norm. By determining whether the physical condition is good or bad based on such a norm, it is possible to determine whether or not the examinee's current physical condition is close to that when he was ill and, if it is, to present a plan for improving physical condition in a more timely fashion.

The determined disparity of the examinee's physical constitution (distance M) may further be compared with another norm to judge whether the disparity of physical constitution is large or not. In that case, the average of the first evaluation value of healthy subjects of the same age can be used as the norm. For example, in a case where the examinee is in his fifties, rather than evaluating the disparity of his physical constitution in comparison with other people in his teens, doing so with other people of the same age range reveals his individual differences in the same age range, and leads to an accurate grasp of a disparity of physical constitution.

As described above, the determiner 53 in the terminal device 51, when determining a disparity of an examinee's physical constitution, takes the distribution of other people's characteristic quantities as the distribution of a population, and uses it as a reference for comparison with the examinee's characteristic quantities. Using the distribution of other people's characteristic quantities as a reference for comparison, it is possible to see a disparity of physical constitution as an individual difference from other people, and thus to easily confirm the effect of a plan for improving a disparity of physical constitution. Moreover, the determiner 53, when determining a change of an examinee's physical condition, takes the distribution of the examinee's own characteristic quantities collected during an evaluation period as the distribution of a population, and uses it as a reference for comparison with the examinee's characteristic quantities. Using the distribution of the examinee's own characteristic quantities as a reference for comparison, it is possible to accurately determine the examinee's physical condition (level of health) while reducing the effect of a disparity of physical constitution and almost eliminating the effect of an individual difference from other people.

Moreover, the determiner 53 finds the distance of the examinee's entered characteristic quantities from the origin $O_1$ of the distribution of other people's characteristic quantities as a first evaluation value, and determines the average of the first evaluation value, which changes with time during the evaluation period, as the disparity of the examinee's physical constitution. Thus, even when the first evaluation value changes or varies during the evaluation period, it is possible to properly determine a disparity of the examinee's physical constitution.

Moreover, the determiner 53 creates the distribution of the examinee's characteristic quantities by collecting them at dates and times at which the first evaluation value, which changes with time during the evaluation period, is equal to or less than a threshold value, finds the distance of the examinee's characteristic quantities from the origin $O_2$ of the above-mentioned distribution as a second evaluation value, and determines the change of the second evaluation value, which changes with time during the evaluation period, as the change of the examinee's physical condition. The above-mentioned distribution of the examinee's characteristic quantities is the distribution of the characteristic quantities for which the first evaluation value is equal to or less than the threshold value, and is considered to indicate a small disparity of physical condition. Accordingly, by finding the second evaluation value by use of the distribution of the examinee's such characteristic quantities as a reference for comparison, and by determining a change of physical condition by use of the second evaluation value, it is possible to determine a change of physical condition while reliably reducing the effect of a disparity of physical constitution.

Moreover, the determiner 53 compares the entered examinee's own characteristic quantities with the distribution of the population's characteristic quantities by use of a Mahalanobis distance. Even with multidimensional characteristic quantities, through comparison with the population's distribution by use of a single measure which specifically is a Mahalanobis distance, it is possible to properly determine a disparity of physical constitution and a change of physical condition.

Moreover, in the embodiment, the distribution of other people's characteristic quantities that is used as the population's distribution when the determiner 53 determines a disparity of physical constitution is the distribution of characteristic quantities of other people in their teens, that is, aged 10 through 19. Young people in their teens are considered to have almost no lifestyle-related disparity of physical constitution. Thus, by use of the above-mentioned distribution, it is possible to accurately determine a disparity of the examinee's physical constitution.

In particular, healthy young people in their teens are considered to have almost no lifestyle-related disparity of physical constitution and to exhibit homogeneous characteristic quantities. Thus, by using the distribution of characteristic quantities of other healthy people aged 10 through 19, it is possible to more accurately determine a disparity of the examinee's physical constitution.

Moreover, the distribution of the examinee's characteristic quantities as the population's distribution that the determiner 53 uses when determining a change of physical condition may instead be the distribution of characteristic quantities of examinees aged 20 or over. An examinee past his teens is liable to have a disparity of physical constitution, and thus the population's distribution is likely to include a disparity of physical constitution. The above-mentioned distribution is effective in a case where a change of the examinee's own physical condition needs to be determined with consideration given to a disparity of physical constitution. In particular, when the above-mentioned distribution is a distribution of characteristic quantities of healthy examinees aged 20 or over, it is possible to determine, with reference to a healthy condition, a change of physical condition with consideration given to a disparity of physical constitution.

Moreover, in the embodiment, the organ as an imaging target is the tongue. In a configuration where an examinee's level of health is determined by imaging the tongue in this way, the above-mentioned effects can be obtained. The organ may be other than the tongue. For example, it is possible to take as an imaging target a region, such as the eyelid, that is prone to develop edema due to poor water metabolism, and to extract characteristic quantities from that region to determine a disparity of physical constitution or a change of physical condition.

Moreover, in the embodiment, as the examinee's characteristic quantities, the color of the tongue, the color of the tongue coating, the shape (thickness) of the tongue, the thickness (shape) of the tongue coating, etc. are used. Thus, the examinee's characteristic quantities include at least one of the color and shape of an organ. By using at least one of the color and shape of an organ as a characteristic quantity, it is possible to determine a disparity of physical constitution and a change of physical condition.

[Adding a Diagnostic Interview]

There is known a method of diagnosing the condition of the human body through a diagnostic interview based on *Kampo* (see, e.g., Katsutoshi Terasawa, "Shorei kara manabu wakan shinryogaku (Japanese Oriental Medicine Learned Through Clinical Cases) (2nd Edition)", Igaku-Shoin Ltd., 1998). The items discussed there may be combined with characteristic quantities from images to derive indices for level of health.

For example, the items discuss there for diagnosing *"Kikyo"* (lack of vital energy) include the following:

1. General weakness
2. Lack of drive
3. Propensity to tiredness
4. Drowsiness during the day
5. Lack of appetite
6. Propensity to catch cold
7. Propensity to be surprised
8. Lack of spirit in the gaze and the voice
9. A whitish pale-pink tongue, or a swollen tongue
10. Weak pulses
11. Weak bowels
12. Atony in the internal organs
13. A flaccid lower abdomen
14. Propensity for diarrhea

Specifically, the items enumerated above needed for a diagnostic interview are displayed on the display 4 of the organ imaging device 1. An examinee takes a tongue image, and in addition operates the operation panel 5 to enter answers to those items. The answers entered are transmitted to the terminal device 51. Based on a comparison of the examinee's characteristic quantities with the population's distribution, and based on the answers transmitted to the terminal device 51 via the operation panel 5, the determiner 53 in the terminal device 51 determines a disparity of the examinee's physical constitution and a change of the examinee's physical condition. For example, when a change of physical condition is determined, the answers to items 1 to 5, 9, 10, and 14 are considered to determine whether or not there is a large change of physical condition. On the other hand, when a disparity of physical constitution is determined, the answers to items 6 to 8 and 11 to 13 are considered to determine whether or not there is a large disparity of physical constitution. The result of determination and a plan for improving physical constitution and physical condition are fed back to the organ imaging device 1, and is displayed on the display 4.

By determining a disparity of physical constitution and a change of physical condition based not only on a comparison between externally entered characteristic quantities and a population's distribution but also on answers returned to diagnostic interview items via the operation panel 5 in this way, it is possible to make a more proper determination with reference to an accumulation of *Kampo* diagnoses and with consideration given to the examinee's own complaints.

[Paid-for Services]

In the level-of-health determining system 50 according to the embodiment, a service may be built that earns fees by providing examinees with diagnosis results and improvement plans. In that case, fees may be varied according to the content presented. For example, in a case where the content presented can be in one of the following patterns - presentation of (a) only change of image characteristic quantities, (b) (a) plus change of physical condition, and (c) (b) plus change of physical constitution, then increasingly high fees can be charged for patterns (a), (b), and (c) in this order. Different fees may be charged according to the time from the taking of an image to the presentation of the result of level-of-health determination.

[One-Dimensional Characteristic Quantity]

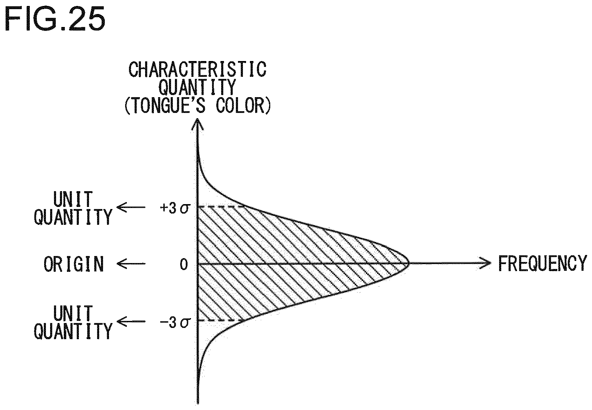

Although the above description deals with a case where characteristic quantities are multidimensional (multivariate), characteristic quantities may be one-dimensional. Fig. 25 shows a frequency distribution in a case where the characteristic quantity is composed of a single variable (e.g., tongue color). The values along the vertical axis are the image data of the tongue color (e.g., R / (R + G + B)). However, the origin corresponds to the average value of the tongue color. By taking the origin of the distribution in Fig. 25 as the origin in Figs. 22 and 23, and taking $\pm 3\sigma$ (where $\sigma$ represents the standard deviation) in Fig. 25 as the unit quantity in Figs. 22 and 23, it is possible to determine a disparity of physical constitution and a change of physical condition.

[Other Examples of Population]

As a reference for comparison in determining a disparity of physical constitution, it is also possible to use a distribution of characteristic quantities extracted from images as discussed below.

(1) Any taken images can be used so long as they are taken images of other healthy people in their teens irrespective of whether they are taken before, at, or after the time of level-of-health determination. For example, in a case where, for an examinee currently aged 50, his level of health when he was aged 40 is to be determined, any images can be used irrespective of whether they were taken before, when, or after the examinee was aged 40. An increased number of kinds of images leads to a clearer determination of level-of health.

(2) Any taken images can be used so long as they are taken images of other unhealthy people in their teens irrespective of whether they are taken before, at, or after the time of level-of-health determination. Through a comparison with other people's characteristic quantities in an unhealthy condition, it is possible to more clearly determine the examinee's condition of health.

(3) Taken images of a healthy or unhealthy examinee himself in his teens may be used. No comparison with other people is then needed, and thus a simple comparison is possible.

As a reference for comparison in determining a change of physical condition, it is possible to use a distribution of characteristic quantities extracted from images as discussed below. Any taken images may be used for any ages so long as they are taken images of a healthy or unhealthy examinee himself irrespective of they are taken before, at, or after evaluation. With an increased number of kinds of images, and through a comparison with a plurality of unhealthy conditions, it is possible to more clearly evaluate the condition of health.

The level-of-health determining device and the level-of-health determining system described above can be expressed as noted below, and provide benefits as noted below.

The level-of-health determining device described above is a level-of-health determining device for determining the level of health of an examinee, and includes: a characteristic quantity acceptor to which a characteristic quantity extracted from a taken image of an organ of an examinee to be used for level-of-health determination is input; and a determiner which compares the examinee's characteristic quantity input via the characteristic quantity acceptor with a distribution of a characteristic quantity of a population and which thereby determines a disparity of the examinee's physical constitution, which corresponds to an individual difference from other people, and a change of the examinee's physical condition. When determining the disparity of the examinee's physical constitution, the determiner uses, as the distribution of the population's characteristic quantity, a distribution of a characteristic quantity of the other people that is created with the other people taken as a target. When determining the change of the examinee's physical condition, the determiner uses, as the distribution of the population's characteristic quantity, a distribution of the examinee's own characteristic quantity collected during an evaluation period for physical condition evaluation.

The determiner compares the examinee's characteristic quantity input via the characteristic quantity acceptor with a distribution of a characteristic quantity of a population, and thereby determines a disparity of the examinee's physical constitution and a change of the examinee's physical condition. Here, when determining the disparity of the examinee's physical constitution, the determiner uses, as the distribution of the population's characteristic quantity, a distribution of a characteristic quantity of the other people. By using a distribution of a characteristic quantity of other people as a reference for comparison, it is possible to see an individual difference from other people, that is, a disparity of the examinee's physical constitution, and thus it is easy to confirm the effect of a plan for improving the disparity of physical constitution.

On the other hand, when determining the change of the examinee's physical condition, the determiner uses, as the distribution of the population's characteristic quantity, a distribution of the examinee's own characteristic quantity collected during an evaluation period. Using a distribution of the examinee's own characteristic quantity as a reference for comparison helps eliminate the effect of an individual difference from other people. Thus, it is possible to accurately determine the examinee's own physical condition (level of health) while reducing the effect of a disparity of physical constitution.

The determiner may find the distance of the examinee's characteristic quantity input via the characteristic quantity acceptor from the origin of the distribution of the other people's characteristic quantity as a first evaluation value, and determine the average of the first evaluation value varying with time during the evaluation period as the disparity of the examinee's physical constitution.

By determining the average of the first evaluation value varying with time as the disparity of the examinee's physical constitution, it is possible to determine a disparity of the examinee's physical constitution irrespective of change and variation of the first evaluation value.

The determiner may collect characteristic quantities at dates and times at which the first evaluation value varying with time during the evaluation period is equal to or less than a threshold value to create a distribution of the examinee's characteristic quantity, find the distance of the examinee's characteristic quantity input via the characteristic quantity acceptor from the origin of the distribution as a second evaluation value, and determine the change of the second evaluation value varying with time during the evaluation period as the change of the examinee's physical condition.

The distribution of the examinee's characteristic quantities obtained by collecting characteristic quantities at dates and times at which the first evaluation value is equal to or less than a threshold value is a distribution of characteristic quantities that are considered to be associated with a small individual difference from other people, that is, a small disparity of physical constitution. Accordingly, by using a distribution of such characteristic quantities as a reference for comparison to find a second evaluation value, and determining a change of physical condition by using the second evaluation value, it is possible to determine a change of physical condition while reliably reducing the effect of a disparity of physical constitution.

The determiner may compare the examinee's characteristic quantity input via the characteristic quantity acceptor with the distribution of the characteristic quantity of the population by use of a Mahalanobis distance. In this case, even when the characteristic quantity input via the characteristic quantity acceptor is multidimensional, it is possible to properly determine a disparity of physical constitution and a change of physical condition.

The distribution of the other people's characteristic quantity may be a distribution of a characteristic quantity of other people aged 10 through 19.

Young people in their teens are considered to have almost no lifestyle-related disparity of physical constitution. Thus, by using a distribution of a characteristic quantity of other people in their teens as the distribution of the characteristic quantity of the population, it is possible, based on that distribution, to accurately determine a disparity of the examinee's physical constitution.

The distribution of the other people's characteristic quantity may be a distribution of a characteristic quantity of other people aged 10 through 19 who are healthy.

Healthy young people in their teens are considered to have almost no lifestyle-related disparity of physical constitution and to exhibit homogeneous characteristic quantities. Thus, by using a distribution of a characteristic quantity of other healthy people aged 10 through 19, it is possible, based on that distribution, to more accurately determine a disparity of the examinee's physical constitution.

The distribution of the examinee's characteristic quantity may be a distribution of a characteristic quantity of an examinee aged 20 or over.

An examinee over age 19 may exhibit a disparity of physical constitution, and this may cause the distribution of the population to include a disparity of physical constitution. Using the above-mentioned distribution is effective in a case where a change of the examinee's own physical condition needs to be determined with consideration given to a disparity of physical constitution.

The above-mentioned distribution of the examinee's characteristic quantity may be a distribution of a characteristic quantity of an examinee aged 20 or over who is healthy.

Despite the possibility of the population's distribution including a disparity of physical constitution, using a distribution of a characteristic quantity of a healthy examinee as a reference for comparison (a distribution of a population's characteristic quantities) makes it possible to determine, with reference to a healthy condition, a change of physical condition with consideration given to a disparity of physical constitution.

The organ may be a tongue. In this case, the above-mentioned effects can be obtained in a configuration that determines an examinee's level of health by taking an image of the tongue.

The examinee's characteristic quantity input via the characteristic quantity acceptor may include information on at least one of the color or the shape of the organ. By using information on at least the color or the shape of an organ as a characteristic quantity, it is possible to determine a disparity of physical constitution and a change of physical condition.

The characteristic quantity acceptor may be configured as a receiver which receives information on the examinee's characteristic quantity transmitted from outside. In this case, the above-mentioned effects can be obtained in a configuration that acquires information on a characteristic quantity of an examinee via a receiver.

The level-of-health determining system described above includes a level-of-health determining device as described above and an organ imaging device that are connected together across a communication network so as to be capable of communicating with each other. The organ imaging device includes: an imager which images an organ of an examinee; a characteristic quantity extractor which extracts from an image taken by the imager a characteristic quantity to be used for level-of-health determination; and a transmitter which transmits information on the examinee's characteristic quantity extracted by the characteristic quantity extractor to the level-of-health determining device. The above-mentioned effects can be obtained in a system in which a characteristic quantity of an examinee to be used for level-of-health determination is acquired by an organ imaging device and is transmitted to a level-of-health determining device.

The organ imaging device may further include a display which displays information on a disparity of physical constitution and a change of physical condition determined by the determiner of the level-of-health determining device. Owing to information on a disparity of the examinee's physical constitution and a change of the examinee's physical condition being displayed on the display, based on the information displayed on the display, the examinee can easily grasp both the effect of improving his physical constitution and his physical condition (level of health).

The organ imaging device may further includes: a display which displays an item needed for a diagnostic interview; and an information acceptor to which an answer to the item displayed on the display is input. The determiner of the level-of-health determining device may determine the disparity of the examinee's physical constitution and the change of the examinee's physical condition based on a comparison between the examinee's characteristic quantity included in the information transmitted from the organ imaging device with the distribution of the population's characteristic quantity, and based on the answer input to the information acceptor and transmitted to the level-of-health determining device.

By determining a disparity of physical constitution and a change of physical condition based not only on a comparison between the transmitted examinee's characteristic quantity with the distribution of the population's characteristic quantity but also on an answer returned to an diagnostic interview item via the information acceptor, it is possible to make a more proper determination.

**Industrial Applicability**

The present invention finds application in systems that determine an examinee's level of health by extracting a characteristic quantity from a taken image of an organ of the examinee.

**List of Reference Signs**

| | |
|---|---|
| 1 | organ imaging device |
| 3 | imager |
| 4 | display |
| 5 | operation panel (information input device) |
| 6 | communicator (transmitter) |
| 16 | characteristic quantity extractor |
| 50 | level-of-health determining system |
| 51 | terminal device (level-of-health determining device) |
| 53 | determiner |
| 54 | communicator (characteristic quantity acceptor, receiver) |

**Claims**

**1.** A level-of-health determining device, comprising:

a characteristic quantity acceptor to which a characteristic quantity extracted from a taken image of an organ of an examinee to be used for level-of-health determination is input; and
a determiner which compares the examinee's characteristic quantity input via the characteristic quantity acceptor with a distribution of a characteristic quantity of a population and which thereby determines a disparity of the examinee's physical constitution, which corresponds to an individual difference from other people, and a change of the examinee's physical condition, wherein

when determining the disparity of the examinee's physical constitution, the determiner uses, as the distribution of the population's characteristic quantity, a distribution of a characteristic quantity of the other people that is created with the other people taken as a target, and
when determining the change of the examinee's physical condition, the determiner uses, as the distribution of the population's characteristic quantity, a distribution of the examinee's own characteristic quantity collected during an evaluation period for physical condition evaluation.

2. The level-of-health determining device of claim 1, wherein
the determiner

finds a distance of the examinee's characteristic quantity input via the characteristic quantity acceptor from an origin of the distribution of the other people's characteristic quantity as a first evaluation value, and
determines an average of the first evaluation value varying with time during the evaluation period as the disparity of the examinee's physical constitution.

3. The level-of-health determining device of claim 2, wherein
the determiner

collects characteristic quantities at dates and times at which the first evaluation value varying with time during the evaluation period is equal to or less than a threshold value to create a distribution of the examinee's characteristic quantity,
finds a distance of the examinee's characteristic quantity input via the characteristic quantity acceptor from an origin of the distribution as a second evaluation value, and
determines a change of the second evaluation value varying with time during the evaluation period as the change of the examinee's physical condition.

4. The level-of-health determining device of any one of claims 1 to 3, wherein
the determiner compares the examinee's characteristic quantity input via the characteristic quantity acceptor with the distribution of the characteristic quantity of the population by use of a Mahalanobis distance.

5. The level-of-health determining device of any one of claims 1 to 4, wherein
the distribution of the other people's characteristic quantity is a distribution of a characteristic quantity of other people aged 10 through 19.

6. The level-of-health determining device of any one of claims 1 to 5, wherein
the distribution of the other people's characteristic quantity is a distribution of a characteristic quantity of other people aged 10 through 19 who are healthy.

7. The level-of-health determining device of any one of claims 1 to 6, wherein
the distribution of the examinee's characteristic quantity is a distribution of a characteristic quantity of an examinee aged 20 or over.

8. The level-of-health determining device of any one of claims 1 to 7, wherein
the distribution of the examinee's characteristic quantity is a distribution of a characteristic quantity of an examinee aged 20 or over who is healthy.

9. The level-of-health determining device of any one of claims 1 to 8, wherein
the organ is a tongue.

10. The level-of-health determining device of any one of claims 1 to 9, wherein
the examinee's characteristic quantity input via the characteristic quantity acceptor includes information on at least one of a color or a shape of the organ.

11. The level-of-health determining device of any one of claims 1 to 10, wherein
the characteristic quantity acceptor comprises a receiver which receives information on the examinee's characteristic quantity transmitted from outside.

12. A level-of-health determining system, comprising the level-of-health determining device of any one of claims 1 to 11 and an organ imaging device connected together across a communication network so as to be capable of communicating with each other, wherein the organ imaging device comprises:

an imager which images an organ of an examinee;
a characteristic quantity extractor which extracts from an image taken by the imager a characteristic quantity to be used for level-of-health determination; and
a transmitter which transmits information on the examinee's characteristic quantity extracted by the characteristic quantity extractor to the level-of-health determining device.

13. The level-of-health determining system of claim 12, wherein the organ imaging device further comprises a display which displays information on a disparity of physical constitution and a change of physical condition determined by the determiner of the level-of-health determining device.

14. The level-of-health determining system of claim 12, wherein the organ imaging device further comprises:

a display which displays an item needed for a diagnostic interview; and
an information acceptor to which an answer to the item displayed on the display is input, wherein

the determiner of the level-of-health determining device determines the disparity of the examinee's physical constitution and the change of the examinee's physical condition based on

a comparison between the examinee's characteristic quantity included in the information transmitted from the organ imaging device with the distribution of the population's characteristic quantity and
the answer input to the information acceptor and transmitted to the level-of-health determining device.

FIG.1

FIG.2

# FIG.3

1

```
                    ┌──────────────────┐        ┌──────────────────┐
              ┌────►│  ILLUMINATION    │───────►│   ILLUMINATOR    │
              │     │   CONTROLLER     │        │                  │
              │     └──────────────────┘        └──────────────────┘
              │              11                          2

              │     ┌──────────────────┐        ┌──────────────────┐
              ├────►│     IMAGING      │───────►│      IMAGER      │
              │     │   CONTROLLER     │        │                  │
              │     └──────────────────┘        └──────────────────┘
              │              12                          3

              │     ┌──────────────────┐        ┌──────────────────┐
              ├────►│     DISPLAY      │───────►│     DISPLAY      │
              │     │   CONTROLLER     │        │                  │
              │     └──────────────────┘        └──────────────────┘
              │              13                          4

              │     ┌──────────────────┐        ┌──────────────────┐
              ├────►│    OPERATION     │───────►│    OPERATION     │
┌──────────┐  │     │   CONTROLLER     │        │      PANEL       │
│ OVERALL  │  │     └──────────────────┘        └──────────────────┘
│CONTROLLER├──┤              14                          5
└──────────┘  │
     20       │     ┌──────────────────┐
              ├────►│ IMAGE PROCESSOR  │
              │     └──────────────────┘
              │              15

              │     ┌──────────────────┐
              │     │  CHARACTERISTIC  │
              ├────►│    QUANTITY      │
              │     │    EXTRACTOR     │
              │     └──────────────────┘
              │              16

              │     ┌──────────────────┐
              ├────►│     STORAGE      │
              │     └──────────────────┘
              │              17

              │     ┌──────────────────┐        ┌──────────────────┐
              ├────►│  COMMUNICATION   │───────►│   COMMUNICATOR   │
              │     │   CONTROLLER     │        │                  │
              │     └──────────────────┘        └──────────────────┘
              │              18                          6

              │     ┌──────────────────┐        ┌──────────────────┐
              └────►│ SOUND DELIVERY   │───────►│     SOUND        │
                    │   CONTROLLER     │        │   DELIVERER      │
                    └──────────────────┘        └──────────────────┘
                             19                          7
```

FIG.4

# FIG.5

TAKEN IMAGE

4

EDGE EXTRACTION FILTER

| | −1 | |
|---|---|---|
| −1 | 4 | −1 |
| | −1 | |

EXTRACTED CONTOUR
LINE

# FIG.6

# FIG.7

METHOD USING DETERMINATION
COEFFICIENT R²

$$y = 5E{-}07x^4 + 6E{-}06x^3 + 0.002x^2 - 0.0629x + 21.213$$

$$R^2 = 0.9942$$

# FIG.8

### METHOD USING COORDINATE DIFFERENCES

$| y_i - f_i |$

# FIG.9

C-C'
SECTION

D-D'
SECTION

# FIG.10

# FIG.11

SPECTRUM DISTRIBUTION ON TONGUE

VISIBLE LIGHT RANGE (400~700nm)

# FIG.12

# FIG.13

# FIG.14

B FREQUENCY DISTRIBUTION (WITHOUT FISSURES)

$\sigma 1 = 13.18$

B FREQUENCY DISTRIBUTION (WITH FISSURES)

$\sigma 2 = 26.78$

# FIG.15

THIN TONGUE

THICK TONGUE

# FIG.16

51

```
                    ┌──────────────┐
               ┌───→│   STORAGE    │
               │    └──────────────┘
               │            └ 52
┌────────────┐ │    ┌──────────────┐
│ CONTROLLER │─┼───→│  DETERMINER  │
└────────────┘ │    └──────────────┘
      └ 55     │            └ 53
               │    ┌──────────────┐
               └───→│ COMMUNICATOR │
                    └──────────────┘
                            └ 54
```

# FIG.17

$$O = \left( \frac{a_1 + a_2}{2} , \frac{b_1 + b_2}{2} \right)$$

FIG.18

PHYSICAL
CONDITION
EVALUATION
VALUE

PATIENT A

EVALUATION
PERIOD

EXAMINEE C

HEALTHY
SUBJECT B

UNIT
QUANTITY
U

P1

ORIGIN O

AGE 20

TIME

FIG.19

PHYSICAL
CONDITION
EVALUATION
VALUE

EVALUATION PERIOD

CHANGE OF
PHYSICAL
CONDITION DURING
PERIOD

UNIT
QUANTITY
U

ORIGIN O

TIME

# FIG.20

START

COLLECT OTHER
PEOPLE'S
(HEALTHY
SUBJECTS')
TONGUE IMAGES
S1-1

EXTRACT
CHARACTERISTIC
QUANTITIES
S1-2

MEASURE-OF-
DISTANCE
CALCULATION 1
S1-3

COLLECT
EXAMINEE'S
TONGUE IMAGES
S1-4

CALCULATE
DISPARITY OF
PHYSICAL
CONSTITUTION
S1-5

COLLECT
EXAMINEE'S
TONGUE IMAGES
(IN GOOD HEALTH)
S2-1

MEASURE-OF-
DISTANCE
CALCULATION 2
S2-2

CALCULATE
CHANGE OF
PHYSICAL
CONDITION
S2-3

UPDATE DATA
S3-1

OUTPUT
DETERMINATION
RESULT
S3-2

UTILIZE DATA
S3-3

END

# FIG.21

PHYSICAL
CONDITION
EVALUATION
VALUE

EVALUATION
PERIOD

DISTANCE
(1)

M

UNIT
QUANTITY
$U_1$

ORIGIN $O_1$

TIME

# FIG.22

PHYSICAL
CONDITION
EVALUATION VALUE
($1_{ST}$ EVALUATION
VALUE)

EVALUATION
PERIOD

AVERAGE
(THRESHOLD VALUE)

CHARACTERISTIC
QUANTITIES EQUAL TO
OR LESS THAN
THRESHOLD VALUE

M

UNIT
QUANTITY $U_1$

ORIGIN $O_1$

TIME

## FIG.23

## FIG.24

# FIG.25

CHARACTERISTIC
QUANTITY
(TONGUE'S COLOR)

UNIT
QUANTITY ← +3σ

ORIGIN ← 0

UNIT
QUANTITY ← −3σ

FREQUENCY

## INTERNATIONAL SEARCH REPORT

| International application No. |
| --- |
| PCT/JP2015/054703 |

**A. CLASSIFICATION OF SUBJECT MATTER**

*G06Q50/22*(2012.01)i, *A61B5/00*(2006.01)i, *G06Q50/24*(2012.01)i, *G06T1/00*
(2006.01)i

According to International Patent Classification (IPC) or to both national classification and IPC

**B. FIELDS SEARCHED**

Minimum documentation searched (classification system followed by classification symbols)
G06Q50/22, A61B5/00, G06Q50/24, G06T1/00

Documentation searched other than minimum documentation to the extent that such documents are included in the fields searched
Jitsuyo Shinan Koho        1922-1996   Jitsuyo Shinan Toroku Koho   1996-2015
Kokai Jitsuyo Shinan Koho   1971-2015   Toroku Jitsuyo Shinan Koho   1994-2015

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2008-54835 A  (Toshiba Corp.),<br>13 March 2008 (13.03.2008),<br>particularly, paragraphs [0005], [0015] to [0026]<br>(Family: none) | 1-14 |
| A | JP 2006-149679 A  (Konica Minolta Holdings, Inc.),<br>15 June 2006 (15.06.2006),<br>particularly, paragraphs [0009] to [0014]<br>(Family: none) | 1-14 |
| A | JP 2001-314376 A  (Research Development Corp. of Japan),<br>13 November 2001 (13.11.2001),<br>particularly, paragraph [0010]<br>(Family: none) | 1-14 |

| ☒ Further documents are listed in the continuation of Box C. | ☐ See patent family annex. |
| --- | --- |

\*    Special categories of cited documents:
"A"   document defining the general state of the art which is not considered    to be of particular relevance
"E"   earlier application or patent but published on or after the international filing date
"L"   document which may throw doubts on priority claim(s) or which is cited to establish the publication date of another citation or other special reason (as specified)
"O"   document referring to an oral disclosure, use, exhibition or other means
"P"   document published prior to the international filing date but later than the priority date claimed

"T"   later document published after the international filing date or priority date and not in conflict with the application but cited to understand the principle or theory underlying the invention
"X"   document of particular relevance; the claimed invention cannot be considered novel or cannot be considered to involve an inventive step when the document is taken alone
"Y"   document of particular relevance; the claimed invention cannot be considered to involve an inventive step when the document is combined with one or more other such documents, such combination being obvious to a person skilled in the art
"&"   document member of the same patent family

| Date of the actual completion of the international search<br>    14 May 2015 (14.05.15) | Date of mailing of the international search report<br>    26 May 2015 (26.05.15) |
| --- | --- |
| Name and mailing address of the ISA/<br>    Japan Patent Office<br>    3-4-3,Kasumigaseki,Chiyoda-ku,<br>    Tokyo 100-8915,Japan | Authorized officer<br><br>Telephone No. |

Form PCT/ISA/210 (second sheet) (July 2009)

**INTERNATIONAL SEARCH REPORT**

International application No.

PCT/JP2015/054703

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2004-113581 A  (Choyo Corp.),<br>15 April 2004 (15.04.2004),<br>entire text; all drawings<br>(Family: none) | 1-14 |
| A | US 2006/0247502 A1  (EASTMAN KODAK CO.),<br>02 November 2006 (02.11.2006),<br>entire text; all drawings<br>& JP 2008-538992 A       & WO 2006/115826 A2<br>& EP 1874188 A2          & CN 101247760 A | 1-14 |

Form PCT/ISA/210 (continuation of second sheet) (July 2009)

REFERENCES CITED IN THE DESCRIPTION

**Patent documents cited in the description**

- JP 4487535 B

- JP 4649965 B

**Non-patent literature cited in the description**

- **KATSUTOSHI TERASAWA.** Shorei kara manabu wakan shinryogaku (Japanese Oriental Medicine Learned Through Clinical Cases). Igaku-Shoin Ltd, 1998